(19) Europäisches Patentamt
European Patent Office
Office européen des brevets

(11) EP 0 975 635 B1

(12) EUROPEAN PATENT SPECIFICATION

(45) Date of publication and mention of the grant of the patent:
**07.05.2003 Bulletin 2003/19**

(21) Application number: **98914274.0**

(22) Date of filing: **23.03.1998**

(51) Int Cl.[7]: **C07D 487/04**, A01N 43/90, C07F 7/08
// (C07D487/04, 249:00, 239:00)

(86) International application number:
**PCT/US98/05615**

(87) International publication number:
**WO 98/046608 (22.10.1998 Gazette 1998/42)**

# (54) FUNGICIDAL TRIFLUOROMETHYLALKYLAMINO-TRIAZOLOPYRIMIDINES

FUNGIZIDE TRIFLUOROMETHYLALKYLAMINO-TRIAZOLOPYRIMIDINE-DERIVATE

TRIFLUOROMETHYLALKYLAMINO-TRIAZOLOPYRIMIDINES FONGICIDES

(84) Designated Contracting States:
**AT BE CH DE DK ES FI FR GB GR IE IT LI LU MC NL PT SE**
Designated Extension States:
**LT LV MK RO SI**

(30) Priority: **14.04.1997 US 843323**

(43) Date of publication of application:
**02.02.2000 Bulletin 2000/05**

(73) Proprietor: **BASF AKTIENGESELLSCHAFT**
**67056 Ludwigshafen (DE)**

(72) Inventors:
- **PEES, Klaus-Juergen**
  **D-55129 Mainz (DE)**
- **KRUMMEL, Guenter**
  **D-55578 Vendersheim (DE)**
- **COTTER, Henry, Van, Tuyl**
  **Trenton, New Jersey 08618 (US)**
- **REHNIG, Annerose**
  **D-55218 Ingelheim (DE)**
- **MAY, Leslie**
  **Berkshire, RG41 1JJ (GB)**
- **PFRENGLE, Waldemar**
  **D-55444 Seibersbach (DE)**
- **ALBERT, Guido**
  **D-55546 Hackenheim (DE)**

(74) Representative: **Langfinger, Klaus Dieter, Dr. et al**
**Director Patents & Licensing,**
**BASF Aktiengesellschaft ZDX/P-C6**
**67056 Ludwigshafen (DE)**

(56) References cited:
**EP-A- 0 071 792**
**EP-A- 0 550 113**
**EP-A- 0 613 900**
**US-A- 5 612 345**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 975 635 B1

## Description

## BACKGROUND OF THE INVENTION

This invention relates to certain triazolopyrimidine compounds, a process for their preparation, compositions containing such compounds, a method for combating a fungus at a locus comprising treating the locus with such compounds and their use as fungicides.

EP-A-0 071 792 claims compounds of the general formula

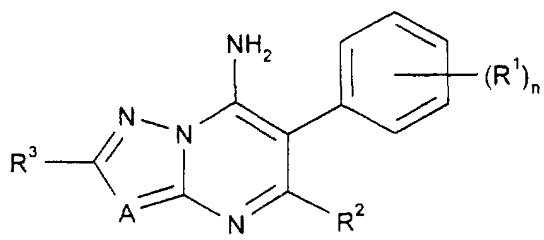

in which $R^1$ represents alkyl, halogen, alkoxy, cyano, cycloalkyl, aryl, aryloxy, arylthio, aralkyl, arylthio, arylalkyl, arylalkyloxy or arylalkylthio each optionally substituted by halogen or alkoxy; or $(R^1)_n$ represents a benzene, indane or tetrahydronaphthalene ring fused with the phenyl ring, aromatic moieties in the above groups being optionally substituted by alkyl, alkoxy, halogen or cyano; n is 1 or 2; $R^2$ and $R^3$ are each hydrogen, alkyl or aryl, A represents a nitrogen atom or a $CR^4$ group, and $R^4$ is as $R^2$ but can also be halogen, cyano or alkoxycarbonyl or together with $R^3$ can form an alkylene chain containing up to two double bonds. The compounds are said to be active against various phytopathogenic fungi, especially those of the phycomycete class. However evidence of fungicidal activity is only provided for these compounds against *Plasmopara viticola*, a member of the oomycete class of fungi.

EP 0 550 113-A2 claims compounds of the general formula

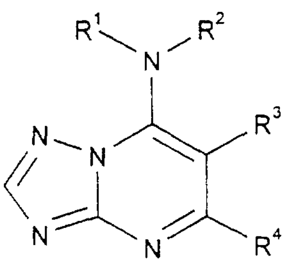

in which $R^1$ represents an optionally substituted alkyl, alkenyl, alkadienyl, cycloalkyl, bicycloalkyl or heterocyclyl group; $R^2$ represents a hydrogen atom or an alkyl group; or $R^1$ and $R^2$ together with the interjacent nitrogen atom represent an optionally substituted heterocyclic ring; $R^3$ represents an optionally substituted aryl group; and $R^4$ represents a hydrogen or halogen atom or a group $-NR^5R^6$ where $R^5$ represents a hydrogen atom or an amino, alkyl, cycloalkyl or bicycloalkyl group and $R^6$ represents a hydrogen atom or an alkyl group. Thus, compounds in which $R^1$ is a trifluoromethylalkyl group are generally embraced by this patent application. However, there is no single compound disclosed in which $R^1$ is a trifluoromethylalkyl group.

Summary of the Invention

The present invention provides a compound of formula I

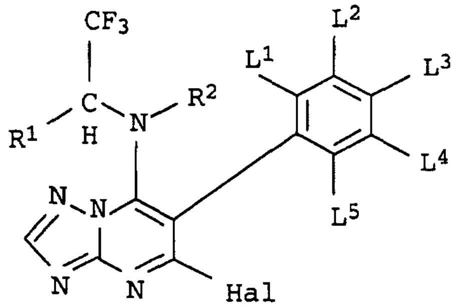

(I)

in which

$R^1$ and $R^2$ each independently represent a hydrogen atom or an optionally substituted alkyl, alkenyl, alkynyl, alkadienyl or phenyl group,
Hal represents a halogen atom,
$L^1$ through $L^5$ each independently represent an hydrogen or halogen atom or an alkyl, alkoxy or nitro group;

in which
the terms alkyl, alkenyl alkadienyl refer to straight or branched chain radicals or moieties having up to 10 carbon atoms; in which each optionally substituted group independently is substituted by one or more halogen atoms or nitro, cyano, hydroxy, alkyl, cycloalkyl, cycloalkenyl, haloalkyl, halocycloalkyl, alkoxy, haloalkoxy, trialkylsilyl, phenyl, halo- or dihalophenyl or pyridyl groups.
The new compounds show an excellent selective fungicidal activity in various crops.
It is an object of the present invention to provide novel, selective fungicidal compounds.
It is also an object of the invention to provide methods for controlling undesired phytopathogenic fungus by contacting said plants with a fungicidally effective amount of the new compounds.
It is another object of the invention to provide selective fungicidal compositions containing the new compounds as active ingredients.
These and other objects and features of the invention will be more apparent from the detailed description set forth hereinbelow, and from the appended claims.

Detailed Description of the preferred empodiments

In has been found that the novel compounds of formula I

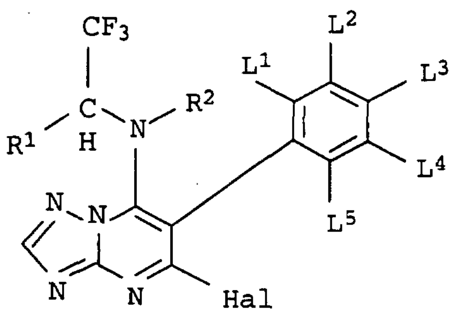

(I)

in which $R^1$ and $R^2$, Hal and $L^1$ through $L^5$ have the meaning given above for formula I show an excellent fungicidal activity against a broad range of phytopathogenic fungi.
In general terms, unless otherwise stated, as used herein the term halogen atom may denote a bromine, iodine, chlorine or fluorine atom, and is especially a bromine, chlorine or fluorine atom.
Hal represents preferably fluorine, chlorine, bromine or iodine, in particular chlorine.
Optionally substituted moieties may be unsubstituted or have from one up to the maximal possible number of substituents. Typically, 0 to 3 substituents are present.

In general terms, unless otherwise stated herein, the terms alkyl, alkenyl, alkynyl, alkadienyl as used herein with respect to a radical or moiety refer to a straight or branched chain radical or moiety. Such radicals have up to 10, in particular up to 6 carbon atoms. Suitably an alkyl moiety has from 1 to 6 carbon atoms, preferably from 1 to 3 carbon atoms. A preferred alkyl moiety is an ethyl or especially a methyl group. Suitably an alkenyl moiety has from 2 to 6 carbon atoms. A preferred alkenyl moiety is allyl or especially a 2-methylallyl group.

The invention especially relates to compounds of the general formula I in which any alkyl part of the groups $R^1$ and/or $R^2$ which may be straight chained or branched, contains preferably up to 9 carbon atoms, more preferably up to 6 carbon atoms, any alkenyl or alkynyl part of the substituents $R^1$ and/or $R^2$ contains preferably up to 9 carbon atoms, more preferably up to 6 carbon atoms, and in which each optionally substituted group independently is substituted by one or more halogen atoms or nitro, cyano, cycloalkyl, preferably $C_{3-6}$ cycloalkyl, cycloalkenyl, preferably $C_{3-6}$ cycloalkenyl, haloalkyl, preferably $C_{1-6}$ haloalkyl, halocycloalkyl, preferably $C_{3-6}$ halocycloalkyl, alkoxy, preferably $C_{1-6}$ alkoxy, haloalkoxy, preferably $C_{1-6}$ haloalkoxy, trialkylsilyl, preferably tri-$C_{1-4}$ alkylsilyl, phenyl, halo- or dihalo-phenyl or pyridyl groups. Any alkyl, alkenyl or alkynyl group may be linear or branched. A halogen atom suitably denotes a fluorine, chlorine or bromine atom.

The invention especially relates to compounds of the general formula I, in which $R^1$ represents a hydrogen atom, a $C_{1-10}$ alkyl or a phenyl group, in particular a hydrogen atom or a methyl group.

Included in the scope of the present invention are (R) and (S) isomers and atropisomers of compounds of general formula I, which have a chiral center or in which the substituents $L^1$ or $L^1$ and $L^2$ are different from $L^5$ or $L^5$ and $L^4$, and the racemates thereof, and salts, N-oxides and acid addition compounds.

Particularly interesting activity has been found in (S)-isomer compounds of general formula I wherein the group $-CH(CF_3)R^1$ is chiral.

Another preferred embodiment of the present invention are the compounds of formula I, wherein $R^2$ represents a hydrogen atom or a $C_{1-10}$ alkyl group or a $C_{3-10}$ alkenyl group.
Those compounds of formula I in which at least one of $R^1$ and $R^2$ represents a hydrogen atom are particularly preferred.

Particularly preferred are compounds of formula I, in which the phenyl group

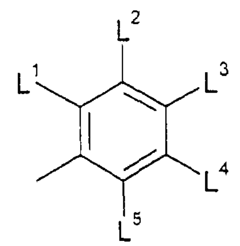

is selected from

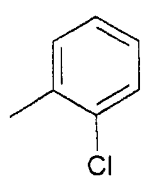 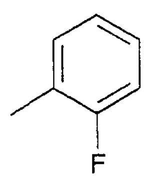 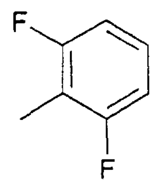

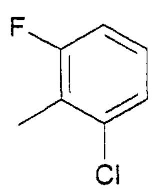 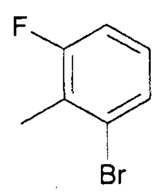 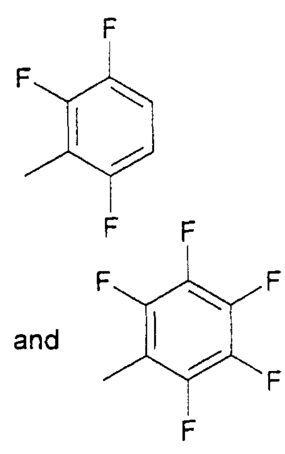

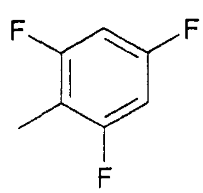 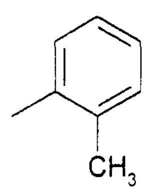 and

The compounds according to general formula I are oils, gums, or, predominantly crystalline solid materials. They are superior through their valuable fungicidal properties, in particular their enhanced systemicity and enhanced fungicitoxity against rice diseases and powdery mildews. For example, they can be used in agriculture or related fields for the control of phytopathogenic fungi such as *Alternaria solani, Botrytis cinerea, Cercospora beticola, Cladosporium herbarum, Corticium rolfsii, Erysiphe graminis, Helminthosporium tritici repentis, Leptosphaeria nodorum, Micronectriella nivalis, Monilinia fructigena, Mycosphaerella ligulicola, Mycosphaerella pinodes, Pyricularia grisea f.sp. oryzae, Rhizoctonia solani, Venturis inaequalis, Uncinula necator* and *Sclerotinia sclerotiorum,* in particular for the control of

*Uncinula necator, Pyricularia grisea f.sp. oryzae* and *Rhizoctonia solani.* The compounds of general formula I according to the invention possess a high fungicidal activity within a wide concentration range and may be used in agriculture without any difficulties.

Moreover, the compounds according to the invention show enhanced residual control of fungi, in particular of grape powdery mildew compared with conventional fungicides.

Good results in terms of control of phythopathogenic fungi are obtained with a compound as defined in formula I wherein:

at least one of $L^1$ and $L^5$ represents a halogen atom; and/or
$R^1$ represents a hydrogen atom or a methyl group.

Especially good results in terms of control of phytopathogenic fungi are obtained by using, for example, the following compounds of formula I:

5-chloro-6-(2-chloro-6-fluorophenyl)-7-(2,2,2-trifluoroethylamino)-[1,2,4]triazolo[1,5-a]pyrimidine, 5-chloro-6-(2,6-difluorophenyl)-7-(2,2,2-trifluoroethylamino)-[1,2,4]triazolo[1,5-a]pyrimidine, 5-chloro-7-(2,2,2-trifluoroethylamino)-6-(2,4,6-trifluorophenyl)-[1,2,4]triazolo[1,5-*a*]pyrimidine, 5-chloro-6-(2-chlorophenyl)-7-(2,2,2-trifluoroethylamino)-[1,2,4]triazolo[1,5-*a*]pyrimidine, 5-chloro-6-(2-fluorophenyl)-7-(2,2,2-trifluoro-ethylamino)-[1,2,4]triazolo[1,5-*a*]pyrimidine, 5-chloro-6-(2-chloro-6-fluorophenyl)-7-[2-(1,1,1-trifluoro)propylamino]-[1,2,4]triazolo[1,5-*a*]pyrimidine, 5-chloro-6-(2,6-difluorophenyl)-7-[2-(1,1,1-trifluoro)propylamino]-[1,2,4]triazolo[1,5-*a*]pyrimidine, 5-chloro-6-(2,46-trifluorophenyl)-7-[2-(1,1,1-trifluoro)propylamino]-[1,2,4]triazolo[1,5-*a*]pyrimidine, 5-chloro-6-(2-methylphenyl)-7-[2-(1,1,1-trifluoro)propylamino]-[1,2,4]triazolo[1,5-*a*]pyrimidine, 5-chloro-6-(2-fluorophenyl)-7-[2-(1,1,1-trifluoro)propylamino]-[1,2,4]triazolo[1,5-*a*]pyrimidine, 5-chloro-6-(2-chlorophenyl)-7-[2-(1,1,1-trifluoro)propylamino]-[1,2,4]triazolo[1,5-*a*]pyrimidine, 5-chloro-6-(2,4,6-trifluorophenyl)-7-[N-(2,2,2-trifluoroethyl)-N-allylamino]-[1,2,4]triazolo[1,5-*a*]pyrimidine, 5-chloro-6-(2-chloro-6-fluorophenyl)-7-[N-(2,2,2-trifluoroethyl)-N-allylamino]-[1,2,4]triazolo[1,5-*a*]pyrimidine, 5-chloro-6-(2-fluorophenyl)-7-[N-(2,2,2-trifluoroethyl)-N-allylamino]-[1,2,4]triazolo[1,5-*a*]pyrimidine, 5-chloro-6-(2,6-difluorophenyl)-7-[N-(2,2,2-trifluoroethyl)-N-allylamino]-[1,2,4]triazolo[1,5-*a*]pyrimidine,5-chloro-6-(2-chlorophenyl)-7-[N-(2,2,2-trifluoroethyl)-N-allylamino]-[1,2,4]triazolo[1,5-*a*]pyrimidine, 5-chloro-6-(2-methylphenyl)-7-[N-(2,2,2-trifluoroethyl)-N-allylamino]-[1,2,4]triazolo[1,5-*a*]pyrimidine, 5-chloro-6-(2-fluorophenyl)-7-[N-(2,2,2-trifluoroethyl)-N-ethylamino]-[1,2,4]triazolo[1,5-*a*]pyrimidine, 5-chloro-6-(2,6-difluorophenyl)-7-[N-(2,2,2-trifluoroethyl)-N-ethylamino]-[1,2,4]triazolo[1,5-*a*]pyrimidine, 5-chloro-6-(2,4,6-trifluorophenyl)-7-[N-(2,2,2-trifluoroethyl)-N-ethylamino]-[1,2,4]triazolo[1,5-*a*]pyrimidine, 5-chloro-6-(2-chloro-6-fluorophenyl)-7-[N-(2,2,2-trifluoroethyl)-N-ethylamino]-[1,2,4]triazolo[1,5-*a*]pyrimidine, 5-chloro-6-(2-chlorophenyl)-7-[N-(2,2,2-trifluoroethyl)-N-ethylamino]-[1,2,4]triazolo[1,5-*a*]pyrimidine, 5-chloro-6-(2-methylphenyl)-7-[N-(2,2,2-trifluoroethyl)-N-ethylamino]-[1,2,4]triazolo[1,5-*a*]pyrimidine, 5-chloro-6-(2-chloro-6-fluorophenyl)-7-[N-(2,2,2-trifluoroethyl)-N-(2-methylpropyl)-amino]-[1,2,4]triazolo[1,5-*a*]pyrimidine, 5-chloro-6-(2,6-difluorophenyl)-7-[N-(2,2,2-trifluoroethyl)-N-(2-methylpropyl)amino]-[1,2,4]triazolo[1,5-*a*]pyrimidine, 5-chloro-6-(2-fluorophenyl)-7-[N-(2,2,2-trifluoroethyl)-N-(2-methylpropyl)amino]-[1,2,4]triazolo[1,5-*a*]pyrimidine, 5-chloro-6-(2,4,6-trifluorophenyl)-7-[N-(2,2,2-trifluoroethyl)-N-(2-methylpropyl)amino]-[1,2,4]triazolo[1,5-*a*]pyrimidine, 5-chloro-6-(2-methylphenyl)-7-[N-(2,2,2-trifluoroethyl)-N-(2-methylpropyl)amino]-[1,2,4]triazolo[1,5-*a*]pyrimidine, 5-chloro-6-(2-fluorophenyl)-7-[N-(2,2,2-trifluoroethyl)-N-methylamino]-[1,2,4]triazolo[1,5-*a*]pyrimidine, 5-chloro-6-(2,6-difluorophenyl)-7-[N-(2,2,2-trifluoroethyl)-N-methylamino]-[1,2,4]triazolo[1,5-*a*]pyrimidine, 5-chloro-6-(2,4,6-trifluorophenyl)-7-[N-(2,2,2-trifluoroethyl)-N-methylamino]-[1,2,4]triazolo[1,5-*a*]pyrimidine, 5-chloro-6-(2-methylphenyl)-7-[N-(2,2,2-trifluoroethyl)-N-methylamino]-[1,2,4]triazolo[1,5-*a*]pyrimidine, 5-chloro-6-(2-fluorophenyl)-7-[N-(2,2,2-trifluoroethyl)-N-isopropylamino]-[1,2,4]triazolo[1,5-*a*]pyrimidine, 5-chloro-6-(2,6-difluorophenyl)-7-[N-(2,2,2-trifluoroethyl)-N-isopropylamino]-[1,2,4]triazolo[1,5-*a*]pyrimidine, 5-chloro-6-(2,4,6-trifluorophenyl)-7-[N-(2,2,2-trifluoroethyl)-N-isopropylamino]-[1,2,4]triazolo[1,5-*a*]pyrimidine, 5-chloro-6-(2-chloro-6-fluorophenyl)-7-[N-(2,2,2-trifluoroethyl)-N-isopropylamino]-[1,2,4]triazolo[1,5-*a*]pyrimidine, 5-chloro-6-(2,6-difluorophenyl)-7-[N-(2,2,2-trifluoroethyl)-N-(1-phenylethyl)amino]-[1,2,4]triazolo[1,5-*a*]pyrimidine, 5-chloro-6-(2-fluorophenyl)-7-[N,N-di-(2,2,2-trifluoroethyl)-amino]-[1,2,4]triazolo[1,5-*a*]pyrimidine, 5-chloro-6-(2-methylphenyl)-7-[N,N-di-(2,2,2-trifluoroethyl)amino]-[1,2,4]triazolo[1,5-*a*]pyrimidine, 5-chloro-6-(2-ch lorophenyl)-7-[N,N-di-(2,2,2-trifluoroethyl)-amino]-[1,2,4]triazolo[1,5-*a*]pyrimidine, 5-chloro-6-(2-chloro-6-fluorophenyl)-7-[N,N-di-(2,2,2-trifluoroethyl)amino]-[1,2,4]triazolo[1,5-*a*]pyrimidine, 5-chloro-6-(2,6-difluorophenyl)-7-[N,N-di-(2,2,2-trifluoroethyl)-amino]-[1,2,4]triazolo[1,5-*a*]pyrimidine, 5-chloro-6-(2,4,6-trifluorophenyl)-7-[N,N-di-(2,2,2-trifluoroethyl) amino]-[1,2,4]triazolo[1,5-*a*]pyrimidine, 5-chloro-6-(2,6-difluorophenyl)-7-[N,-(2,2,2-trifluoroethyl)-N-(1,2-dimethylpropyl)amino]-[1,2,4]triazolo[1,5-*a*]pyrimidine, 5-chloro-6-(2,4,6-trifluorophenyl)-7-[N,-(2,2,2-trifluoroethyl)-N-(trimethylsilylmethyl)amino]-[1,2,4]triazolo[1,5-*a*]pyrimidine, 5-chloro-6-(2-bromo-5-chlorophenyl)-7-[2-(1,1,1-trifluoro)propylamino]-[1,2,4]triazolo[1,5-*a*]pyrimidine, 5-chloro-6-(2-chlorophenyl)-7-(2,2,2-trifluoroethylamino)-[1,2,4]triazolo[1,5-*a*]pyrimidine, 5-chloro-6-(4-chlorophenyl)-7-(2,2,2-trifluoroethylamino)-[1,2,4]triazolo[1,5-*a*]pyrimidine, 5-chloro-6-(4-bromophenyl)-7-(2,2,2-trifluoroethylamino)-[1,2,4]triazolo[1,5-*a*]pyrimidine,5-chloro-6-(4-methoxyphenyl)-7-(2,2,2-trifluoroethylamino)-[1,2,4]triazolo[1,5-*a*]pyrimidine, 5-chloro-6-(4-nitrophenyl)-7-(2,2,2-trifluoroethylamino)-[1,2,4]triazolo[1,5-*a*]pyrimidine, 5-chloro-6-(2-fluorophenyl)-7-(1-phenyl-2,2,2-trifluoroethylamino)-[1,2,4]triazolo[1,5-*a*]pyrimidine, 5-chloro-6-(2-chloro-6-fluorophenyl)-7-[2-(1,1,1-trifluoro)butylamino]-[1,2,4]triazolo[1,5-*a*]pyrimidine, 5-chloro-6-(2-chloro-6-fluorophenyl)-7-(1-phenyl-2,2,2-trifluoroethylamino)-[1,2,4]triazolo[1,5-*a*]pyrimidine, 5-chloro-6-(2-fluorophenyl)-7-[2-(1,1,1-trifluoro)butylamino]-[1,2,4]triazolo[1,5-*a*]pyrimidine, 5-chloro-6-(2,6-difluorophenyl)-7-(1-phenyl-2,2,2-trifluoroethylamino)-[1,2,4]triazolo[1,5-*a*]pyrimidine, 5-chloro-6-(2,4,6-trifluorophenyl)-7-(1-phenyl-2,2,2-trifluoroethylamino)-[1,2,4]triazolo[1,5-*a*]pyrimidine, 5-chloro-6-(2,4,6-trifluorophenyl)-7-[2-(1,1,1-trifluoro)butylamino]-[1,2,4]triazolo[1,5-*a*]pyrimidine, 5-chloro-6-(2,6-difluorophenyl)-7-[2-(1,1,1-trifluoro)butylamino]-[1,2,4]triazolo[1,5-*a*]pyrimidine, 5-chloro-6-(2-chlorophenyl)-7-[2-(1,1,1-trifluoro)butylamino]-[1,2,4]triazolo[1,5-*a*]pyrimidine, 5-chloro-6-(2-chlorophenyl)-7-(1-phenyl-2,2,2-trifluoroethylamino)-[1,2,4]triazolo[1,5-*a*]pyrimidine, 5-chloro-6-(2,4-difluorophenyl)-7-(2,2,2-trifluoroethylamino)-[1,2,4]triazolo[1,5-*a*]pyrimidine, 5-chloro-6-(2,4,6-trifluorophenyl)-7-[2-(1,1,1-trifluoro)-3-methylbutylamino]-[1,2,4]triazolo[1,5-*a*]pyrimidine, 5-chloro-6-(2,6-difluoro-4-methoxyphenyl)-7-[2-(1,1,1-trifluoro)propylamino]-[1,2,4]triazolo[1,5-*a*]pyrimidine.

The present invention further provides a process for the preparation of a compound of formula I as defined above which comprises

treating a compound of the general formula II

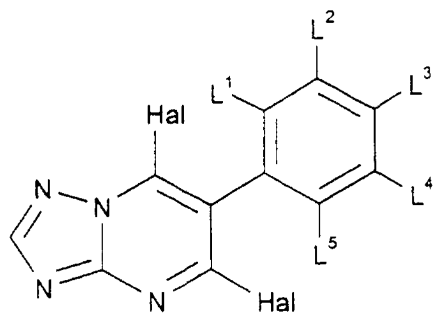

(II)

in which

$L^1$ through $L^5$ and Hal are as defined in any one of the preceding claims; with an amine or amide of the general formula III

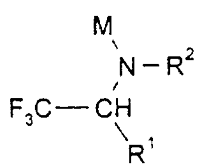

(III)

in which

$R^1$ and $R^2$ are as defined hereinbefore,

M represents a hydrogen atom or a free or complexed metal atom, preferably selected from the group consisting of Li, Na, K, Zn and Cu,

to produce a compound of formula I

Compounds of formula II are known e.g. from EP 0 550 113 and are conventionally prepared by reacting 3-amino-1,2,4-triazole with 2-phenyl-substituted malonic acid ester of formula IV,

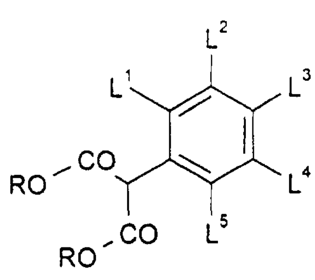

(IV)

wherein R represents alkyl, under alkaline conditions, preferably using high boiling tertiary amines as for example tri-n-butylamine.

The resulting 5,7-dihydroxy-6-phenyltriazolopyrimidines are subsequently treated with a halogenating agent, preferably with a brominating or chlorinating agent, such as phosphorus oxybromide or phosphorus oxychloride, neat or in the presence of a solvent. The reaction is suitably carried out at a temperature in the range from 0 °C to 150 °C, the preferred reaction temperature being from 80 °C to 125 °C.

The reaction between the 5,7-dihalo-6-phenyltriazolopyrimidines of formula II and the amine or amide of formula III is conveniently carried out in the presence of a solvent. Suitable solvents include ethers, such as dioxane, diethyl ether and, especially, tetrahydrofuran, halogenated hydrocarbons such as dichloromethane and aromatic hydrocarbons, for example toluene. The reaction is suitably carried out at a temperature in the range from 0 °C to 70 °C, the preferred reaction temperature being from 10 °C to 35 °C. It is also preferred that the reaction is carried out in the presence of a base. Suitable bases include tertiary amines, such as triethylamine, and inorganic bases, such as potassium carbonate or sodium carbonate. Alternatively, an excess of the compound of formula III may serve as a base.

The compounds according to the invention may also be obtained by reacting a 7-amino-5-halo-6-phenyltriazolopyrimidine with a trifluoroalkanoic acid or a reactive derivative thereof, in particular with trifluoroacetic acid anhydride, in the presence of a base and subsequent reduction of the resulting trifluoroalkanoic amide.

The amines of formula III, wherein M represents a hydrogen atom, are well-known in the literature or commercially available or may be prepared analogously to methods that are known per se. The amides of formula III, wherein M represents a metal atom are, as a rule, obtained from the corresponding amines (M = hydrogen) by reaction with an alkyl lithium compound optionally followed by a transmetallation reaction.

Due to excellent activity, the compounds of formula I may be used in cultivation of all plants where infection by phytopathogenic fungi is not desired, e.g. cereals, solanaceous crops, vegetables, legumes, apples, vine.

The compounds of general formula I have been found to have fungicidal activity. Accordingly, the invention further provides a fungicidal composition which comprises an active ingredient, which is at least one compound of formula I as defined above, and one or more carriers. A method of making such a composition is also provided which comprises bringing a compound of formula I as defined above into association with the carrier(s). Such a composition may contain a single active ingredient or a mixture of several active ingredients of the present invention. It is also envisaged that different isomers or mixtures of isomers may have different levels or spectra of activity and thus compositions may comprise individual isomers or mixtures of isomers.

A composition according to the invention preferably contains from 0.5% to 95% by weight (w/w) of active ingredient.

A carrier in a composition according to the invention is any material with which the active ingredient is formulated to facilitate application to the locus to be treated, which may for example be a plant, seed, soil, or water in which a plant grows, or to facilitate storage, transport or handling. A carrier may be a solid or a liquid, including material which is normally a gas but which has been compressed to form a liquid.

The compositions may be manufactured into e.g. emulsion concentrates, solutions, oil in water emulsions, wettable powders, soluble powders, suspension concentrates, dusts, granules, water dispersible granules, micro-capsules, gels, tablets and other formulation types by well-established procedures. These procedures include intensive mixing and/or milling of the active ingredients with other substances, such as fillers, solvents, solid carriers, surface active compounds (surfactants), and optionally solid and/or liquid auxiliaries and/or adjuvants. The form of application such as spraying, atomizing, dispersing or pouring may be chosen like the compositions according to the desired objectives and the given circumstances.

Solvents may be aromatic hydrocarbons, e.g. Solvesso® 200, substituted naphthalenes, phthalic acid esters, such as dibutyl or dioctyl phthalate, aliphatic hydrocarbons, e.g. cyclohexane or paraffins, alcohols and glycols as well as their ethers and esters, e.g. ethanol, ethyleneglycol mono- and dimethyl ether, ketones such as cyclohexanone, strongly polar solvents such as N-methyl-2-pyrrolidone, or γ-butyrolactone, higher alkyl pyrrolidones, e.g. n-octylpyrrolidone or cyclohexylpyrrolidone, epoxidized plant oil esters, e.g. methylated coconut or soybean oil ester and water. Mixtures of different liquids are often suitable.

Solid carriers, which may be used for dusts, wettable powders, water dispersible granules, or granules, may be mineral fillers, such as calcite, talc, kaolin, montmorillonite or attapulgite. The physical properties may be improved by addition of highly dispersed silica gel or polymers. Carriers for granules may be porous material, e.g. pumice, kaolin, sepiolite, bentonite; non-sorptive carriers may be calcite or sand. Additionally, a multitude of pre-granulated inorganic or organic materials may be used, such as dolomite or crushed plant residues.

Pesticidal compositions are often formulated and transported in a concentrated form which is subsequently diluted by the user before application. The presence of small amounts of a carrier which is a surfactant facilitates this process of dilution. Thus, preferably at least one carrier in a composition according to the invention is a surfactant. For example, the composition may contain at two or more carriers, at least one of which is a surfactant.

Surfactants may be nonionic, anionic, cationic or zwitterionic substances with good dispersing, emulsifying

and wetting properties depending on the nature of the compound according to general formula I to be formulated. Surfactants may also mean mixtures of individual surfactants.

The compositions of the invention may for example be formulated as wettable powders, water dispersible granules, dusts, granules, tablets, solutions, emulsifiable concentrates, emulsions, suspension concentrates and aerosols. Wettable powders usually contain 5 to 90% w/w of active ingredient and usually contain in addition to solid inert carrier, 3 to10% w/w of dispersing and wetting agents and, where necessary, 0 to 10% w/w of stabilizer(s) and/or other additives such as penetrants or stickers. Dusts are usually formulated as a dust concentrate having a similar composition to that of a wettable powder but without a dispersant, and may be diluted in the field with further solid carrier to give a composition usually containing 0.5 to 10% w/w of active ingredient. Water dispersible granules and granules are usually prepared to have a size between 0.15 mm and 2.0 mm and may be manufactured by a variety of techniques. Generally, these types of granules will contain 0.5 to 90% w/w active ingredient and 0 to 20% w/w of additives such as stabilizer, surfactants, slow release modifiers and binding agents. The so-called "dry flowables" consist of relatively small granules having a relatively high concentration of active ingredient. Emulsifiable concentrates usually contain, in addition to a solvent or a mixture of solvents, 1 to 80% w/v active ingredient, 2 to 20% w/v emulsifiers and 0 to 20% w/v of other additives such as stabilizers, penetrants and corrosion inhibitors. Suspension concentrates are usually milled so as to obtain a stable, non-sedimenting flowable product and usually contain 5 to 75% w/v active ingredient, 0.5 to 15% w/v of dispersing agents, 0.1 to 10% w/v of suspending agents such as protective colloids and thixotropic agents, 0 to 10% w/v of other additives such as defoamers, corrosion inhibitors, stabilizers, penetrants and stickers, and water or an organic liquid in which the active ingredient is substantially insoluble; certain organic solids or inorganic salts may be present dissolved in the formulation to assist in preventing sedimentation and crystalization or as antifreeze agents for water.

Aqueous dispersions and emulsions, for example compositions obtained by diluting the formulated product according to the invention with water, also lie within the scope of the invention.

Of particular interest in enhancing the duration of the protective activity of the compounds of this invention is the use of a carrier which will provide slow release of the pesticidal compounds into the environment of a plant which is to be protected.

The biological activity of the active ingredient can also be increased by including an adjuvant in the spray dilution. An adjuvant is defined here as a substance which can increase the biological activity of an active ingredient but is not itself significantly biologically active. The adjuvant can either be included-in the formulation as a coformulant or carrier, or can be added to the spray tank together with the formulation containing the active ingredient.

As a commodity the compositions may preferably be in a concentrated form whereas the end user generally employs diluted compositions. The compositions may be diluted to a concentration down to 0.001% of active ingredient. The doses usually are in the range from 0.01 to 10 kg a.i./ha.

Examples of formulations according to the invention are:

| Emulsion Concentrate (EC) | | |
|---|---|---|
| Active Ingredient | Compound of Example 8 | 30 % (w/v) |
| Emulsifier(s) | Atlox® 4856 B / Atlox® 4858 B [1)](mixture containing calcium alkyl aryl sulfonate, fatty alcohol ethoxylates and light aromatics / mixture containing calcium alkyl aryl sulfonate, fatty alcohol ethoxylates and light aromatics) | 5 % (w/v) |
| Solvent | Shellsol® A [2)] (mixture of $C_9$ - $C_{10}$ aromatic hydrocarbons) | to 1000 ml |
| Suspension Concentrate (SC) | | |
| Active Ingredient | Compound of Example 8 | 50 % (w/v) |
| Dispersing agent | Soprophor® FL[3)] (polyoxyethylene polyaryl phenyl ether phosphate amine salt) | 3 % (w/v) |
| Antifoaming agent | Rhodorsil® 422 [3)] (nonionic aqueous emulsion of polydimethylsiloxanes) | 0.2 % (w/v) |
| Structure agent | Kelzan® S [4)] (Xanthan gum) | 0.2 % (w/v) |
| Antifreezing agent | Propylene glycol | 5 % (w/v) |

1) commercially available from ICI Surfactants
2) commercially available from Deutsche Shell AG
3) commercially available from Rhône-Poulenc
4) commercially available from Kelco Co.

(continued)

| | | |
|---|---|---|
| Emulsion Concentrate (EC) | | |
| Suspension Concentrate (SC) | | |
| Biocidal agent | Proxel® 5)<br>(aqueous dipropylene glycol solution containing 20% 1,2-benisothiazolin-3-one) | 0.1 %<br>(w/v) |
| Water | | to 1000 ml |
| Wettable Powder (WP) | | |
| Active Ingredient | Compound of Example 8 | 60 % (w/w) |
| Wetting agent | Atlox® 4995 1) (polyoxyethylene alkyl ether) | 2 % (w/w) |
| Dispersing agent | Witcosperse® D-60 6) (mixture of sodium salts of condensed naphthalene sulfonic acid and alkylarylpolyoxy acetates | 3 % (w/w) |
| Carrier / Filler | Kaolin | 35 % (w/w) |
| Water Dispersible Granules (WG) | | |
| Active Ingredient | Compound of Example 8 | 50 % (w/w) |
| Dispersing / Binding agent | Witcosperse® D-450 6)<br>(mixture of sodium salts of condensed naphthalene sulfonic acid and alkyl sulfonates) | 8 % (w/w) |
| Wetting agent | Morwet® EFW 6) (formaldehyde condensation product) | 2 % (w/w) |
| Antifoaming agent | Rhodorsil® EP 6703 3) (encapsulated silicone) | 1 % (w/w) |
| Disintegrant | Agrimer® ATF 7) (cross-linked homopolymer of N-vinyl-2-pyrrolidone) | 2 % (w/w) |
| Carrier / Filler | Kaolin | 35 % (w/w) |

5) commercially available from Zeneca
6) commercially available from Witco
7) commercially available from International Speciality Products

The compositions of this invention can also comprise other compounds having biological activity, e.g. compounds having similar or complementary pesticidal activity or compounds having plant growth regulating, fungicidal or insecticidal activity. These mixtures of pesticides can have a broader spectrum of activity than the compound of general formula I alone. Furthermore, the other pesticide can have a synergistic effect on the pesticidal activity of the compound of general formula I.

The other fungicidal compound can be, for example, one which is also capable of combating diseases of cereals (e.g. wheat) such as those caused by *Erysipha, Puccinia, Septoria, Gibberella* and *Helminthosporium* spp., seed and soil borne diseases and downy and powdery mildews on vines, early and late blight on solanaceous crops, and powdery mildew and scab on apples etc. These mixtures of fungicides can have a broader spectrum of activity than the compound of general formula I alone. Furthermore, the other fungicide can have a synergistic effect on the fungicidal activities of the compound of general formula I.

Examples of the other fungicidal compounds are anilazine, azoxystrobin, benalaxyl, benomyl, bethoxazin, binapacryl, bitertanol, blasticidin S, Bordeaux mixture, bromuconazole, bupirimate, captafol, captan, carbendazim, carboxin, carpropamid, chlorbenzthiazon, chlorothalonil, chlozolinate, copper-containing compounds such as copper oxychloride, and copper sulfate, cycloheximide, cymoxanil, cypofuram, cyproconazole, cyprodinil, dichlofluanid, dichlone, dichloran, diclobutrazol, diclocymet, diclomezine, diethofencarb, difenoconazole, diflumetorim, dimethirimol, dimethomorph, diniconazole, dinocap, ditalimfos, dithianon, dodemorph, dodine, edifenphos, epoxiconazole, etaconazole, ethirimol, etridiazole, famoxadone, fenapanil, fenarimol, fenbuconazole, fenfuram, fenhexamid, fenpiclonil, fenpropidin, fenpropimorph, fentin, fentin acetate, fentin hydroxide, ferimzone, fluazinam, fludioxonil, flumetover, fluquinconazole, flusilazole, flusulfamide, flutolanil, flutriafol, folpet, fosetyl-aluminium, fuberidazole, furalaxyl, furametpyr, guazatine, hexaconazole, imazalil, iminoctadine, ipconazole, iprodione, isoprothiolane, kasugamycin, kitazin P, kresoxim-methyl, mancozeb, maneb, mepanipyrim, mepronil, metalaxyl, metconazole, methfuroxam, myclobutanil, neoasozin, nickel dimethyldithiocarbamate, nitrothalisopropyl, nuarimol, ofurace, organo mercury compounds, oxadixyl, oxycarboxin, penconazole, pencycuron, phenazineoxide, phthalide, polyoxin D, polyram, probenazole, prochloraz, procymidione, propamocarb, propiconazole, propineb, pyrazophos, pyrifenox, pyrimethanil, pyroquilon, pyroxyfur, quinomethionate, quinoxyfen, quintozene, spiroxamine, SSF-126, SSF-129, streptomycin, sulfur, tebuconazole, tecloftalame, tecnazene, tetraconazole, thiabendazole, thifluzamide, thiophanate-methyl, thiram, tolclofosmethyl, tolylfluanid, triadimefon, triadimenol, triazbutil, triazoxide, tricyclazole, tridemorph, triflumizole, triforine, triticonazole, validamycin A, vinclozolin, XRD-563, zarilamid, zineb, ziram

In addition, the co-formulations according to the invention may contain at least one compound of formula I and any of the following classes of biological control agents such as viruses, bacteria, nematodes, fungi, and other microorganisms which are suitable to control insects, weeds or plant diseases or to induce host resistance in the plants. Examples of such biological control agents are: *Bacillus thuringiensis, Verticillium lecanii, Autographica californica NPV, Beauvaria bassiana, Ampelomyces quisqualis, Bacilis subtilis, Pseudomonas chlororaphis, Pseudomonas fluorescens, Steptomyces griseoviridis* and *Trichoderma harzianum*.

Moreover, the co-formulations according to the invention may contain at least one compound of formula I and a chemical agent that induces the systemic acquired resistance in plants such as for example nicotinic acid or derivatives thereof or BION.

The compounds of general formula I can be mixed with soil, peat or other rooting media for the protection of the plants against seed-borne, soil-borne or foliar fungal diseases.

The invention still further provides the use as a fungicide of a compound of the general formula I as defined above or a composition as defined above, and a method for combating fungus at a locus, which comprises treating the locus, which may be for example plants subject to or subjected to fungal attack, seeds of such plants or the medium in which such plants are growing or are to be grown, with such a compound or composition.

The present invention is of wide applicability in the protection of crop and ornamental plants against fungal attack. Typical crops which may be protected include vines, grain crops such as wheat and barley, rice, sugar beet, top fruit, peanuts, potatoes, vegetables and tomatoes. The duration of the protection is normally dependent on the individual compound selected, and also a variety of external factors, such as climate, whose impact is normally mitigated by the use of a suitable formulation.

The following examples further illustrate the present invention. It should be understood, however, that the invention is not limited solely to the particular examples given below.

## Example 1

5-Chloro-6-(2-chloro-6-fluorophenyl)-7-N-(2,2,2-trifluoroethylamino)-1,2,4-triazolo[1.5a]pyrimidine

A mixture of 2,2,2-trifluoroethylamine (4.2 mmoles) and dichloromethane (10 ml) is added to a mixture of 5,7-dichloro-6-(2-chloro-6-fluorophenyl)-1,2,4-triazolo[1.5a]pyrimidine (1.4 mmoles) and dichloromethane (30 ml) under stirring. The reaction mixture is stirred 16 hours at room temperature, subsequently washed two times with 1 N hydrochloric acid and once with water. The organic layer is separated, dried with anhydrous sodium sulfate and the solvent is evaporated under reduced pressure. Treatment of the resulting light brown oil with *tert.*-butyl methyl ether (50 ml) yields beige crystals having a melting point of 194-197 °C.

## Examples 2-69

The following examples (Table I; structure and melting point) are synthesized analogously to Example 1.

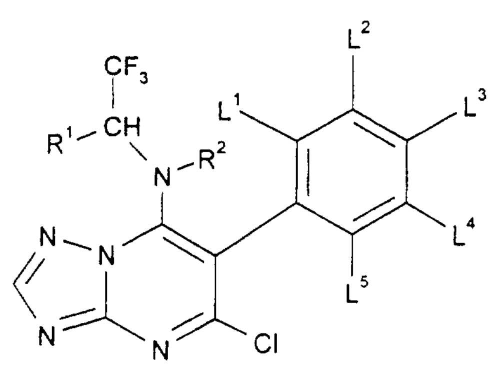

| ***Example*** | *$R^1$* | *$R^2$* | *$L^1$* | *$L^2$* | *$L^3$* | *$L^4$* | *$L^5$* | ***melting point** (° **C)*** |
|---|---|---|---|---|---|---|---|---|
| **2** | H | H | Cl | H | H | H | H | 165 |
| **3** | H | H | F | H | F | H | F | 195 |
| **4** | H | H | F | H | H | H | H | 180 |
| **5** | H | H | F | H | H | H | F | 137 |
| **6** | $CH_3$ | H | Cl | H | H | H | F | 165-176 |
| **7** | $CH_3$ | H | F | H | H | H | F | 166-167 |
| **8** | $CH_3$ | H | F | H | F | H | F | 184-185 |
| **9** | $CH_3$ | H | $CH_3$ | H | H | H | H | 149-151 |
| **10** | $CH_3$ | H | F | H | H | H | H | 128-130 |
| **11** | $CH_3$ | H | Cl | H | H | H | H | 129-130 |
| **12** | H | allyl | F | H | F | H | F | 104-105 |
| **13** | H | allyl | Cl | H | H | H | F | 145-146 |
| **14** | H | allyl | F | H | H | H | H | 128 |
| **15** | H | allyl | F | H | H | H | F | 109-111 |
| **16** | H | allyl | Cl | H | H | H | H | 129-130 |
| **17** | H | allyl | $CH_3$ | H | H | H | H | 123-130 |
| **18** | H | ethyl | F | H | H | H | H | 136-139 |
| **19** | H | ethyl | F | H | H | H | F | 164-166 |
| **20** | H | ethyl | F | H | F | H | F | 133-134 |
| **21** | H | ethyl | Cl | H | H | H | F | 199-202 |
| **22** | H | ethyl | Cl | H | H | H | H | 150-158 |
| **23** | H | ethyl | $CH_3$ | H | H | H | H | 174-178 |
| **24** | H | 2-methylpropyl | Cl | H | H | H | F | 175 |
| **25** | H | 2-methylpropyl | F | H | H | H | F | 154-155 |
| **26** | H | 2-methylpropyl | F | H | H | H | H | 144 |
| **27** | H | 2-methylpropyl | F | H | F | H | F | 133-134 |
| **28** | H | 2-methylpropyl | $CH_3$ | H | H | H | H | 154-155 |
| **29** | H | methyl | F | H | H | H | H | 142-143 |
| **30** | H | methyl | F | H | H | H | F | 175-177 |
| **31** | H | methyl | F | H | F | H | F | 163 |
| **32** | H | methyl | F | H | H | H | Cl | 178-180 |
| **33** | H | methyl | $CH_3$ | H | H | H | H | 147-149 |
| **34** | H | isopropyl | F | H | H | H | F | 147-150 |
| **35** | H | isopropyl | F | H | H | H | Cl | 178-183 |
| **36** | H | isopropyl | F | H | F | H | F | 154-158 |
| **37** | H | isopropyl | F | H | H | H | H | 167-170 |
| **38** | H | 1-phenylethyl | F | H | H | H | H | oil |
| **39** | H | 2,2,2-trifluoroethyl | Cl | H | H | H | H | 212-213 |
| **40** | H | 2,2,2-trifluoroethyl | $CH_3$ | H | H | H | H | 223-226 |
| **41** | H | 2,2,2-trifluoroethyl | F | H | H | H | H | 185-186 |
| **42** | H | 2,2,2-trifluoroethyl | F | H | H | H | Cl | 234-237 |
| **43** | H | 2,2,2-trifluoroethyl | F | H | H | H | F | 208-210 |
| **44** | H | 2,2,2-trifluoroethyl | F | H | F | H | F | 177-179 |
| **45** | H | 1,2-dimethylpropyl | F | H | H | H | F | 154-158 |
| **46** | H | trimethylsilylmethyl | F | H | F | H | F | 85 |
| **47** | $CH_3$ | methyl | Br | H | H | Cl | H | 160-169 |
| **48** | H | H | H | H | Cl | H | H | 170 |
| **49** | H | H | H | H | Br | H | H | 176-177 |

(continued)

| ***Example*** | *$R^1$* | *$R^2$* | *$L^1$* | *$L^2$* | *$L^3$* | *$L^4$* | *$L^5$* | ***melting point** (°**C)*** |
|---|---|---|---|---|---|---|---|---|
| **50** | H | H | H | H | $OCH_3$ | H | H | 183-185 |
| **51** | H | H | H | H | $NO_2$ | H | H | 237 |
| **52** | $C_6H_5$ | H | F | H | H | H | H | 114 |
| **53** | $C_2H_5$ | H | F | H | H | H | Cl | 162 |
| **54** | $C_6H_5$ | H | F | H | H | H | Cl | 108 |
| **55** | $C_2H_5$ | H | F | H | H | H | H | 133 |
| **56** | $C_6H_5$ | H | F | H | H | H | F | 148 |
| **57** | $C_6H_5$ | H | F | H | F | H | F | 86 |
| **58** | $C_2H_5$ | H | F | H | F | H | F | 177 |
| **59** | $C_2H_5$ | H | F | H | F | H | F | 171 |
| **60** | $C_2H_5$ | H | Cl | H | H | H | H | oil |
| **61** | $C_6H_5$ | H | Cl | H | H | H | H | oil |
| **62** | H | H | F | H | F | H | H | 181 |
| **63** | i-$C_3H_7$ | H | F | H | F | H | F | 104 |
| **64** | H | H | F | H | $OCH_3$ | H | F | oil |
| **65** | H | H | F | H | H | H | Br | 187 |
| **66** | $CH_3$ | H | F | H | H | H | Br | 184-185 |
| **67** | H | H | F | F | H | H | F | 183 |
| **68** | CH3 | H | F | F | H | H | F | 149 |
| **69** | H | H | F | F | F | F | F | oil |

**Biological Investigations**

**A.** Determination of Minimum Inhibitory Concentration by Test Compounds in the Serial Dilution Test with Various Phytopathogenic Fungi

The MIC (Minimum Inhibitory Concentration) value, which indicates the lowest concentration of the active ingredient in the growth medium which causes a total inhibition of myecelial growth, is determined by serial dilution tests using Microtiter plates with 24 or 48 wells per plate. The dilution of the test compounds in the nutrient solution and the distribution to the wells is carried out by a TECAN RSP 5000 Robotic Sample Processor. The following test compound concentrations are used: 0.05, 0.10, 0.20, 0.39, 0.78, 1.56, 3.13, 6.25, 12.50, 25.00, 50.00 and 100.00 mg/ml. For preparation of the nutrient solution, V8 vegetable juice (333 ml) is mixed with calcium carbonate (4.95 g), centrifuged, the supernatant (200 ml) diluted with water (800 ml) and autoclaved at 121 °C for 30 min.

The respective inocula (*Altemaria solani,* ALTESO; *Botrytis cinerea,* BOTRCI; *Leptosphaeria nodorum,* LEPTNO; *Phytophthora infestans*, PHYTIN; *Magnaporthe grisea f. sp. oryzae*, PYRIOR; *Pyrenophora teres,* PYRNTE; *Rhizoctonia solani*, RHIZSO;) are added into the wells as spore suspensions (50 ml; $5x10^5$/ml) or agar slices (6 mm) of an agar culture of the fungus.

After 6-12 days incubation at suitable temperatures (18-25°C), the MIC values are determined by visual inspection of the plates (Table II; n. t. = not tested).

Table II

| Ex. No. | ALTESO | BOTRCI | LEPTNO | PHYTIN | PYRIOR | PYRNTE | RHISZO |
|---|---|---|---|---|---|---|---|
| 1 | 3.13 | 1.56 | 6.25 | 12.5 | 0.78 | 6.25 | 6.25 |
| 2 | 12.5 | 6.25 | 50 | 12.5 | 0.78 | 12.5 | 25 |
| 3 | 1.56 | 1.56 | 6.25 | 25 | 0.2 | 6.25 | 6.25 |
| 6 | 3.13 | 12.5 | 25 | > 100 | 3.13 | 25 | 6.25 |
| 7 | 1.56 | 25 | 50 | > 100 | 0.78 | 12.5 | 6.25 |
| 8 | 0.78 | 3.13 | 3.13 | > 100 | 0.78 | 6.25 | 3.13 |
| 9 | 25 | 25 | 12.5 | > 100 | 3.13 | > 100 | 12.5 |

Table II (continued)

| Ex. No. | ALTESO | BOTRCI | LEPTNO | PHYTIN | PYRIOR | PYRNTE | RHISZO |
|---|---|---|---|---|---|---|---|
| 10 | 6.25 | > 100 | 50 | 25 | 1.56 | > 100 | 12.5 |
| 11 | 3.13 | 6.25 | 25 | 100 | 0.39 | 6.25 | 6.25 |
| 12 | 0.39 | 0.78 | 3.13 | > 100 | 0.04 | 3.13 | 0.78 |
| 13 | 3.13 | 3.13 | > 100 | > 100 | 0.1 | > 100 | 1.56 |
| 15 | 1.56 | 1.56 | 6.25 | > 100 | 0.04 | 6.25 | 1.56 |
| 16 | 1.56 | 6.25 | 12.5 | > 100 | 0.39 | 50 | 50 |
| 19 | 0.78 | 1.56 | > 100 | > 100 | 0.04 | > 100 | 0.78 |
| 20 | 0.78 | 0.78 | 3.13 | > 100 | 0.04 | 1.56 | 0.78 |
| 27 | 0.2 | 0.2 | 0.39 | > 100 | 0.04 | > 100 | 3.13 |
| 30 | 3.13 | 12.5 | > 100 | > 100 | 0.78 | 25 | > 100 |
| 31 | 0.78 | 3.13 | 50 | > 100 | 0.1 | 12.5 | 1.56 |
| 46 | 0.78 | 6.25 | > 100 | 25 | 1.56 | 25 | > 100 |
| 47 | 12.5 | 12.5 | 25 | > 100 | 1.56 | > 100 | 50 |
| 49 | 12.5 | 25 | 50 | 100 | 0.78 | 25 | 50 |
| 53 | 6.25 | 12.5 | > 100 | > 100 | 1.56 | > 100 | > 100 |
| 55 | 3.13 | 12.5 | 50 | > 100 | 1.56 | > 100 | > 100 |
| 56 | 3.13 | 12.5 | > 100 | > 100 | 1.56 | 3.13 | > 100 |
| 57 | 50 | > 100 | > 100 | > 100 | 0.78 | 100 | > 100 |
| 59 | 6.25 | 12.5 | 25 | > 100 | 0.39 | > 100 | 6.25 |
| 60 | 3.13 | 25 | 25 | 25 | 1.56 | 25 | 25 |
| 61 | 0.39 | 1.56 | 1.56 | 100 | 0.04 | 6.25 | 12.5 |
| standard * | 12.5 | 12.5 | 100 | > 100 | 50 | 50 | 100 |

* 5-Chloro-6-(2-chlorophenyl)-7-N-ethylamino-1,2,4-triazolo[1.5a]pyrimidine has been used as standard; this compound corresponds to the compound of example 2, in which the 7-(2,2,2-trifluoroethyl)amino group has been replaced by an ordinary 7-ethylamino group.

**B.** Determination of residual control of grape powdery mildew on vine leaves Method for Evaluation of the compounds of formula I to control powdery mildew on grapes (*Uncinula necator)*

Test plants

Cuttings of cultivar Müller-Thurgau were grown in the greenhose at temperatures between 18 °C and 25 °C and 50 to 70 % relative humidity. When 6 to 8 leaves had developed the plants were cut back to 3 - 4 equally sized leaves. Plants were cultivated in pots containing FLORAGAD as a substrate.

Application

Three to four plants per treatment were used. Application of the compounds of formula I was carried out 3 days before infection in the prophylactic tests. The test plants were sprayed to run off in a spray cabinett using 20 ml of spray wash. The compounds of formula I were dissolved in acetone at a concentration of 0.5 %. The stock solution was diluted with water to give the final concentrations. Formulated fungicides were also diluted with water prior to application.

Infection

The plants were artificially infected with conidia of *Uncinula necator* by dusting spores from freshly sporulating grape leaves from the *Uncinula necator* stock culture over the test plants. The spores were allowed to settle on the leaves for 1 hour. The plants remained in the greenhouse without additional light at temperatures between 16 °C and 30 °C for 24 hours.

Evaluation

Evaluation was carried out 21 days after infection by assessing the percentage of infected leaf area of each

of the 4 treated leaves. The activity in % was calculated using the ABBOTT formula:

$$\% \text{ activity} = 100 - \frac{\% \text{ infection in treated}}{\% \text{ infection in untreated}} \times 100$$

The results of this evaluation are shown in Table III:

Table III

| *Uncinula necator* on Vines | | |
|---|---|---|
| **Example No.** | **Rate (ppm a.i.)** | **Efficacy (%)** |
| **3** | 25 | 100 |
| | 12.5 | 100 |
| | 6.25 | 100 |
| | 3.13 | 91 |
| **2** | 25 | 38 |
| | 12.5 | 19 |
| | 6.25 | 5 |
| | 3.13 | 0 |
| **8** | 25 | 100 |
| | 12.5 | 100 |
| | 6.25 | 100 |
| | 3.13 | 97 |
| **standard *** | 25 | 5 |
| | 12.5 | 11 |
| | 6.25 | 2 |
| | 3.13 | 0 |

* The same compound as in the investigations regarding the MIC values has been used as standard.

**C.** Foliar Systemicity

C-1: Grape variety Müller-Thurgau

PATHOGEN: Uncinula necator

TEST PROCEDURE:

1. Grape cuttings are grown in 8 cm diameter plastic in the greenhouse.
2. Formulated compounds are applied to a young fully expanded leaf in a transverse band using an airbrush with a 0.5 mm circular nozzle at an air pressure of 500 mbar. The band is sprayed onto the lower leaf surface, perpendicular to the leaf axis using a cardboard mask with a 5-mm wide slit. The location of the band is marked on the upper leaf surface using a permanent marker and is typically 4 cm from the leaf apex. After application, the plants are not moved until the bands are dry.
3. After treated plants have dried, they are moved to the greenhouse and kept there for 2 days to allow for movement of the compounds. The plants are maintained with bottom watering.
4. Two days after application, the grape plants are inoculated by dusting them with powdery mildew conidia in the greenhouse. Sporulating diseased leaves from stock culture plants are brushed with a brush in the air over the test plants so that the conidia can settle onto the upper leaf surfaces of the test plants. Evaluations are made 12 - 14 days after inoculation.

C-2 Wheat Powdery Mildew (WPM):

HOST: Wheat (Triticum aestivum L.) variety Kanzler

PATHOGEN: Erysiphe graminis DC. f.sp. tritici E. Marchal

TEST PROCEDURE:

1. Wheat seed (8/pot) is planted in 8 cm diameter plastic pots in the greenhouse.
2. When the primary leaf is fully expanded, the plants are cut back to four in each pot of which two are marked with a permanent marker 5 cm below the leaf tip on the upper leaf surface. Thus there are two band-treated and two untreated plants in each pot.
3. A pipette is used to apply 5 µl of the formulated compound in a band on the lower leaf surface opposite the mark. The application band should cover the whole leaf width. After application, the plants are not moved until the bands are dry (half an hour or so later).
4. After treated plants have dried, they are moved to the greenhouse and kept there for 2 days to allow for movement of the compounds. The plants are maintained with bottom watering.
5. Two days after application, the plants are inoculated by dusting them with powdery mildew conidia in the greenhouse. Evaluations are made 7 - 8 days after inoculation. Alternatively, plants are inoculated with Puccinia recondita (wheat leaf rust pathogen) and evaluations made 7 - 8 days thereafter.

Evaluation

Three types of compound movement are assessed by evaluating disease in three areas of each band-treated leaf.

Translaminar movement: The percent disease area is assessed for the translaminar band area (marked area of the upper leaf surface directly opposite where band was applied on the lower leaf surface; width of band approximately 5 mm). Translaminar disease control is then calculated using the following formula:

$$\%\ \text{disease control} = 100 - \frac{\%\ \text{disease in treated plants}}{\%\ \text{disease in untreated plants}} \times 100$$

Distal movement and proximal movement: The distal and proximal disease-free zones on the upper leaf surface are measured in mm. The distal direction is from the band toward the leaf apex and the proximal direction is from the band toward the leaf base. The percent of the disease-free zone relative to the entire distance between the band and leaf apex or base is calculated (for Grape 40 mm equals 100%). If disease is noticeably lighter in the distal or proximal area this is also noted.

FORMULATION AND CONTROLS:

1. The compounds are formulated in a solvent/surfactant system consisting of 5% acetone and 0.05% Tween 20 in deionized water. Compounds are dissolved in acetone prior to dilution with Tween water. Formulated compounds are prepared using deionized water. Compounds are typically tested at 400 ppm.
2. Three kinds of controls are included:

Plants band-treated with the solvent/surfactant solution and inoculated (Solvent Blank).
Untreated plants which are inoculated (Inoculated Control).

The results of this evaluation are shown in Table IV, in which the diseases have been abbreviated as follows:

| | |
|---|---|
| Wheat powdery mildew | WPM |
| Grape powdery mildew | GPM |
| Wheat leaf rust | WLR |

Table IV

| Foliar Systemicity | | | | |
|---|---|---|---|---|
| **Ex. No.** | Disease | Proximal Movement (mm from band) | Distal Movement (mm from band) | Translaminar Activity ( % ) |
| 1 | WPM | 3 | 43 | 100 |
| | GPM | 6 | 50 | 100 |
| | WLR | 5 | 48 | 100 |
| **2** | GPM | 8 | 36 | 100 |
| | WLR | 4 | 21 | 100 |
| **3** | GPM | 7 | 50 | 100 |
| | WLR | 4 | 23 | 100 |
| **4** | GPM | 7 | 32 | 100 |
| | WLR | 3 | 6 | 100 |
| **Standard[2]** | GPM | 2 | 6 | 100 |
| | WLR | 4 | 8 | 100 |

5-chloro-7-(4-methylpiperidin-1-yl)-6-(2-chloro-6-fluorophenyl)-[1,2,4]triazolo[1,5-*a*]pyrimidine which is disclosed in US patent no. 5,593,996 has been used as standard[2].

## Claims

**1.** A compound of the general formula I

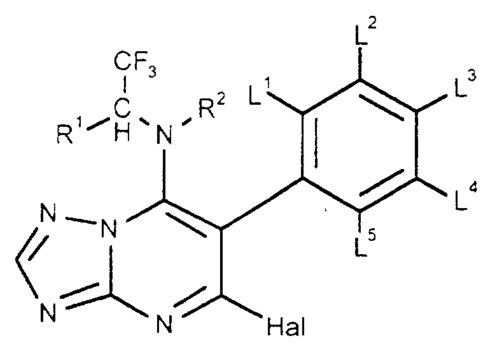

(I)

in which

$R^1$ and $R^2$ each independently represent a hydrogen atom or an optionally substituted alkyl, alkenyl, alkynyl, alkadienyl or phenyl group,
Hal represents a halogen atom
$L^1$ through $L^5$ each independently represent an hydrogen or halogen atom or an alkyl, alkoxy or nitro group

in which
the terms alkyl, alkenyl, alkynyl alkadienyl refer to straight or branched chain radicals or moieties having up to 10 carbon atoms;
in which each optionally substituted group independently is substituted by one or more halogen atoms or nitro, cyano, hydroxy, alkyl, cycloalkyl, cycloalkenyl, haloalkyl, halocycloalkyl, halocycloalkyl, alkoxy, haloalkoxy, trialkylsilyl, phenyl, halo- or dihalo-phenyl or pyridyl groups.

**2.** A compound according to Claim 1 in which at least one of $L^1$ and $L^5$ represents a halogen atom.

**3.** A compound according to Claim 1 or 2 in which $R^1$ represents hydrogen atom or a methyl group.

**4.** A compound according to any of the preceding Claims in which $R^2$ represents a hydrogen or a $C_{1-10}$ alkyl group.

**5.** A compound according to any of the preceding Claims in which at least one of $R^1$ and $R^2$ represents a hydrogen atom.

**6.** The following compounds of formula I:

5-chloro-6-(2-chloro-6-fluorophenyl)-7-(2,2,2-trifluoroethylamino)-[1,2,4]triazolo[1,5-*a*]pyrimidine
5-chloro-6-(2,6-difluorophenyl)-7-(2,2,2-trifluoroethylamino)-[1,2,4]triazolo[1,5-*a*]pyrimidine
5-chloro-7-(2,2,2-trifluoroethylamino)-6-(2,46-trifluorophenyl)-[1,2,4]triazolo[1,5-*a*]pyrimidine
5-chloro-6-(2-chlorophenyl)-7-(2,2,2-trifluoroethylamino)-[1,2,4]triazolo[1,5-*a*]pyrimidine
5-chloro-6-(2-fluorophenyl)-7-(22,2-trifluoroethylamino)-[1,2,4]triazolo[1,5-*a*]pyrimidine
5-chloro-6-(2-chloro-6-fluorophenyl)-7-[2-(1,1,1-trifluoro)propylamino]-[1,2,4]triazolo[1,5-*a*]pyrimidine
5-chloro-6-(2,6-difluorophenyl)-7-[2-(1,1,1-trifluoro)propylamino]-[1,2,4]triazolo[1,5-*a*]pyrimidine
5-chloro-6-(2,4,6-trifluorophenyl)-7-[2-(1,1,1-trifluoro)propylamino]-[1,2,4]triazolo[1,5-*a*]pyrimidine
5-chloro-6-(2-methylphenyl)-7-[2-(1,1,1-trifluoro)propylamino]-[1,2,4]triazolo[1,5-*a*]pyrimidine
5-chloro-6-(2-fluorophenyl)-7-[2-(1,1,1-trifluoro)propylamino]-[1,2,4]triazolo[1,5-*a*]pyrimidine
5-chloro-6-(2-chlorophenyl)-7-[2-(1,1,1-trifluoro)propylamino]-[1,2,4]triazolo[1,5-*a*lpyrimidine
5-chloro-6-(2,4,6-trifluorophenyl)-7-[N-(2,2,2-trifluoroethyl)-N-allylamino]-[1,2,4]triazolo[1,5-*a*]pyrimidine
5-chloro-6-(2-chloro-6-fluorophenyl)-7-[N-(2,2,2-trifluoroethyl)-N-allylamino]-[1,2,4]triazolo[1,5-*a*]pyrimidine
5-chloro-6-(2-fluorophenyl)-7-[N-(2,2,2-trifluoroethyl)-N-allylamino]-[1,2,4]triazolo[1,5-*a*]pyrimidine
5-chloro-6-(2,6-difluorophenyl)-7-[N-(2,2,2-trifluoroethyl)-N-allylamino]-[1,2,4]triazolo[1,5-*a*]pyrimidine
5-chloro-6-(2-chlorophenyl)-7-[N-(2,2,2-trifluoroethyl)-N-allylamino]-[1,2,4]triazolo[1,5-*a*]pyrimidine
5-chloro-6-(2-methylphenyl)-7-[N-(2,2,2-trifluoroethyl)-N-allylamino]-[1,2,4]triazolo[1,5-*a*]pyrimidine
5-chloro-6-(2-fluorophenyl)-7-[N-(2,2,2-trifluoroethyl)-N-ethylamino]-[1,2,4]triazolo[1,5-*a*]pyrimidine
5-chloro-6-(2,6-difluorophenyl)-7-[N-(2,2,2-trifluoroethyl)-N-ethylamino]-[1,2,4]triazolo[1,5-*a*]pyrimidine
5-chloro-6-(2,4,6-trifluorophenyl)-7-[N-(2,2,2-trifluoroethyl)-N-ethylamino]-[1,2,4]triazolo[1,5-*a*]pyrimidine
5-chloro-6-(2-chloro-6-fluorophenyl)-7-[N-(2,2,2-trifluoroethyl)-N-ethylamino]-[1,2,4]triazolo[1,5-*a*]pyrimidine
5-chloro-6-(2-chlorophenyl)-7-[N-(2,2,2-trifluoroethyl)-N-ethylamino]-[1,2,4]triazolo[1,5-*a*]pyrimidine
5-chloro-6-(2-methylphenyl)-7-[N-(2,2,2-trifluoroethyl)-N-ethylamino]-[1,2,4]triazolo[1,5-*a*]pyrimidine
5-chloro-6-(2-chloro-6-fluorophenyl)-7-[N-(2,2,2-trifluoroethyl)-N-(2-methylpropyl)amino]-[1,2,4]triazolo [1,5-*a*]pyrimidine
5-chloro-6-(2,6-difluorophenyl)-7-[N-(2,2,2-trifluoroethyl)-N-(2-methylpropyl)amino]-[1,2,4]triazolo[1,5-*a*]pyrimidine
5-chloro-6-(2-fluorophenyl)-7-[N-(2,2,2-trifluoroethyl)-N-(2-methylpropyl)-amino]-[1,2,4]triazolo[1,5-*a*]pyrimidine
5-chloro-6-(2,4,6-trifluorophenyl)-7-[N-(2,2,2-trifluoroethyl)-N-(2-methylpropyl)amino]-[1,2,4]triazolo[1,5-*a*] pyrimidine
5-chloro-6-(2-methylphenyl)-7-[N-(2,2,2-trifluoroethyl)-N-(2-methylpropyl)-amino]-[1,2,4]triazolo[1,5-*a*]pyrimidine
5-chloro-6-(2-fluorophenyl)-7-[N-(2,2,2-trifluoroethyl)-N-methylamino]-[1,2,4]triazolo[1,5-*a*]pyrimidine
5-chloro-6-(2,6-difluorophenyl)-7-[N-(2,2,2-trifluoroethyl)-N-methylamino]-[1,2,4]triazolo[1,5-*a*]pyrimidine
5-chloro-6-(2,4,6-trifluorophenyl)-7-[N-(2,2,2-trifluoroethyl)-N-methylamino]-[1,2,4]triazolo[1,5-*a*]pyrimidine
5-chloro-6-(2-methylphenyl)-7-[N-(2,2,2-trifluoroethyl)-N-methylamino]-[1,2,4]triazolo[1,5-*a*]pyrimidine
5-chloro-6-(2-fluorophenyl)-7-[N-(2,2,2-trifluoroethyl)-N-isopropylamino]-[1,2,4]triazolo[1,5-*a*]pyrimidine
5-chloro-6-(2,6-difluorophenyl)-7-[N-(2,2,2-trifluoroethyl)-N-isopropylamino]-[12,4]triazolo[15-*a*]pyrimidine
5-chloro-6-(2,4,6-trifluorophenyl)-7-[N-(2,2,2-trifluoroethyl)-N-isopropylamino]-[1,2,4]triazolo[1,5-*a*]pyrimidine
5-chloro-6-(2-chloro-6-fluorophenyl)-7-[N-(2,2,2-trifluoroethyl)-N-isopropylamino]-[1,2,4]triazolo[1,5-*a*]pyrimidine
5-chloro-6-(2,6-difluorophenyl)-7-[N-(2,2,2-trifluoroethyl)-N-(1-phenylethyl)amino]-[1,2,4]triazolo[1,5-*a*]pyrimidine
5-chloro-6-(2-fluorophenyl)-7-[N,N-di-(2,2,2-trifluoroethyl)amino]-[1,2,4]triazolo[1,5-*a*]pyrimidine
5-chloro-6-(2-methylphenyl)-7-[N,N-di-(2,2,2-trifluoroethyl)amino]-[1,2,4]triazolo[1,5-*a*]pyrimidine
5-chloro-6-(2-chlorophenyl)-7-[N,N-di-(2,2,2-trifluoroethyl)amino]-[1,2,4]triazolo[1,5-*a*]pyrimidine
5-chloro-6-(2-chloro-6-fluorophenyl)-7-[N,N-di-(2,2,2-trifluoroethyl)amino]-[1,2,4]triazolo[1,5-*a*]pyrimidine

5-chloro-6-(2,6-difluorophenyl)-7-[N,N-di-(2,2,2-trifluoroethyl)amino]-[1,2,4]triazolo[1,5-*a*]pyrimidine
5-chloro-6-(2,4,6-trifluorophenyl)-7-[N,N-di-(2,2,2-trifluoroethyl)amino]-[1,2,4]triazolo[15-*a*]pyrimidine
5-chloro-6-(2,6-difluorophenyl)-7-[N,-(2,2,2-trifluoroethyl)-N-(1,2-dimethylpropyl)amino]-[1,2,4]triazolo[1,5-*a*]pyrimidine
5-chloro-6-(2,4,6-trifluorophenyl)-7-[N,-(2,2,2-trifluoroethyl)-N-(trimethylsilylmethyl)amino]-[1,2,4]triazolo[1,5-a]pyrimidine
5-chloro-6-(2-bromo-5-chlorophenyl)-7-[2-(1,1,1-trifluoro)propylamino]-[1,2,4]triazolo[1,5-a]pyrimidine
5-chloro-6-(2-chlorophenyl)-7-(2,2,2-trifluoroethylamino)-[1,2,4]triazolo[1,5-a]pyrimidine
5-chloro-6-(4-chlorophenyl)-7-(2,2,2-trifluoroethylamino)-[1,2,4]triazolo[1,5-a]pyrimidine
5-chloro-6-(4-bromophenyl)-7-(2,2,2-trifluoroethylamino)-[1,2,4]triazolo[1,5-a]pyrimidine
5-chloro-6-(4-methoxyphenyl)-7-(2,2,2-trifluoroethylamino)-[1,2,4]triazolo[1,5-a]pyrimidine
5-chloro-6-(4-nitrophenyl)-7-(2,2,2-trifluoroethylamino)-[1,2,4]triazolo[1,5-a]pyrimidine
5-chloro-6-(2-fluorophenyl)-7-(1-phenyl-2,2,2-trifluoroethylamino)-[1,2,4]triazolo[1,5-a]pyrimidine
5-chloro-6-(2-chloro-6-fluorophenyl)-7-[2-(1,1,1-trifluoro)butylamino]-[1,2,4]triazolo[1,5-a]pyrimidine
5-chloro-6-(2-chloro-6-fluorophenyl)-7-(1-phenyl-2,2,2-trifluoroethylamino)-[1,2,4]triazolo[1,5-a]pyrimidine
5-chloro-6-(2-fluorophenyl)-7-[2-(1,1,1-trifluoro)butylamino]-[1,2,4]triazolo[1,5-a]pyrimidine
5-chloro-6-(2,6-difluorophenyl)-7-(1-phenyl-2,2,2-trifluoroethylamino)-[1,2,4]triazolo[1,5-a]pyrimidine
5-chloro-6-(2,4,6-trifluorophenyl)-7-(1-phenyl-2,22-trifluoroethylamino)-[1,2,4]triazolo[1,5-ajpyrimidine
5-chloro-6-(2,4,6-trifluorophenyl)-7-[2-(1,1,1-trifluoro)butylamino]-[1,2,4]triazolo[1,5-a]pyrimidine
5-chloro-6-(2,6-difluorophenyl)-7-[2-(1,1,1-trifluoro)butylamino]-[1,2,4]triazolo[1,5-a]pyrimidine
5-chloro-6-(2-chlorophenyl)-7-[2-(1,1,1-trifluoro)butylamino]-[1,2,4]triazolo[1,5-a]pyrimidine
5-chloro-6-(2-chlorophenyl)-7-(1-phenyl-2,2,2-trifluoroethylamino)-[1,2,4]triazolo[1,5-a]pyrimidine
5-chloro-6-(2,4-difluorophenyl)-7-(2,2,2-trifluoroethylamino)-[1,2,4]triazolo[1,5-a]pyrimidine
5-chloro-6-(2,4,6-trifluorophenyl)-7-[2-(1,1,1-trifluoro)-3-methylbutylamino]-[1,2,4]triazolo[1,5-a]pyrimidine
5-chloro-6-(2,6-difluoro-4-methoxyphenyl)-7-[2-(1,1,1-trifluoro)propylamino]-[1,2,4]triazolo[1,5-a]pyrimidine.

**7.** A process for the preparation of a compound of formula I as defined in any one of the preceding claims which comprises
treating a compound of the general formula II

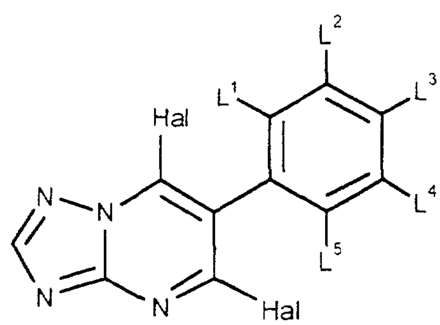

(II)

in which
$L^1$ through $L^5$ and Hal are as defined in any one of the preceding claims;
with an amine of the general formula III

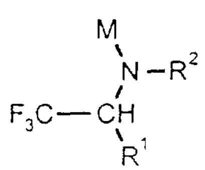

(III)

in which
$R^1$ and $R^2$ are as defined in any one of the preceding claims,
M represents a hydrogen atom or a free or complexed metal atom,
to produce a compound of formula I.

**8.** A fungicidal composition which comprises a carrier, and as active agent, at least one compound of formula I as defined in any one of Claims 1 to 6.

**9.** A method of combating phytopathogenic fungus at a locus which comprises treating the locus with a compound of formula I as defined in any one of Claims 1 to 6 or with a composition as defined in Claim 8.

**10.** The use as a fungicide of a compound of formula I as defined in any one of Claims 1 to 6 or a composition as defined in Claim 8.

**Patentansprüche**

**1.** Verbindung der allgemeinen Formel I

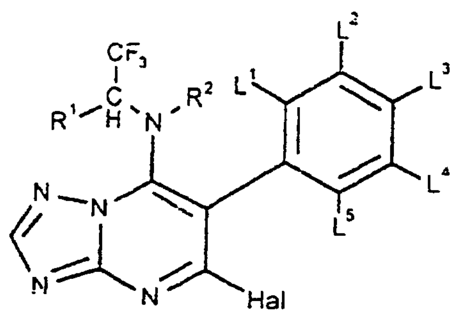

(I)

in der

$R^1$ und $R^2$ jeweils unabhängig ein Wasserstoffatom oder eine gegebenenfalls substituierte Alkyl-, Alkenyl-, Alkinyl-, Alkadienyl- oder Phenylgruppe bedeuten,
Hal ein Halogenatom bedeutet,
$L^1$ bis $L^5$ jeweils unabhängig ein Wasserstoff- oder Halogenatom oder eine Alkyl-, Alkoxy- oder Nitrogruppe bedeuten,

wobei
sich die Ausdrücke Alkyl, Alkenyl, Alkinyl und Alkadienyl auf gerade- oder verzweigtkettige Reste oder Molekülteile mit bis zu 10 Kohlenstoffatomen beziehen;
wobei jede gegebenenfalls substituierte Gruppe unabhängig durch ein oder mehrere Halogenatome oder durch Nitro-, Cyano-, Hydroxy-, Alkyl-, Cycloalkyl-, Cycloalkenyl-, Halogenalkyl-, Halogencycloalkyl-, Alkoxy-, Halogenalkoxy-, Trialkylsilyl-, Phenyl-, Halogen- oder Dihalogenphenyl- oder Pyridylgruppen substituiert ist.

**2.** Verbindung nach Anspruch 1, in der mindestens eine der Gruppen $L^1$ und $L^5$ ein Halogenatom bedeutet.

**3.** Verbindung nach Anspruch 1 oder 2, in der $R^1$ ein Wasserstoffatom oder eine Methylgruppe bedeutet.

**4.** Verbindung nach einem der vorhergehenden Ansprüche, in der $R^2$ ein Wasserstoffatom oder eine $C_{1-10}$Alkylgruppe bedeutet.

**5.** Verbindung nach einem der vorhergehenden Ansprüche, in der mindestens einer der Reste $R^1$ und $R^2$ ein Wasserstoffatom bedeutet.

**6.** Die folgenden Verbindungen der Formel I:

5-Chlor-6-(2-chlor-6-fluorphenyl)-7-(2,2,2-trifluorethylamino)-[1,2,4]triazolo[1,5-*a*]pyrimidin
5-Chlor-6-(2,6-difluorphenyl)-7-(2,2,2-trifluorethylamino)-[1,2,4]triazolo[1,5-*a*]pyrimidin
5-Chlor-7-(2,2,2-trifluorethylamino)-6-(2,4,6-trifluorphenyl)-[1,2,4]triazolo[1,5-*a*]pyrimidin
5-Chlor-6-(2-chlorphenyl)-7-(2,2,2-trifluorethylamino)-[1,2,4]triazolo[1,5-*a*]pyrimidin

5-Chlor-6-(2-fluorphenyl)-7-(2,2,2-trifluorethylamino)-[1,2,4]triazolo[1,5-*a*]pyrimidin
5-Chlor-6-(2-chlor-6-fluorphenyl)-7-[2-(1,1,1-trifluor)propylamino]-[1,2,4]triazolo[1,5-*a*]pyrimidin
5-Chlor-6-(2,6-difluorphenyl)-7-[2-(1,1,1-trifluor)propylamino]-[1,2,4]triazolo[1,5-*a*]pyrimidin
5-Chlor-6-(2,4,6-trifluorphenyl)-7-[2-(1,1,1-trifluor)propylamino]-[1,2,4]triazolo[1,5-*a*]pyrimidin
5-Chlor-6-(2-methylphenyl)-7-[2-(1,1,1-trifluor)propylamino]-[1,2,4]triazolo[1,5-*a*]pyrimidin
5-Chlor-6-(2-fluorphenyl)-7-[2-(1,1,1-trifluor)propylamino]-[1,2,4]triazolo[1,5-*a*]pyrimidin
5-Chlor-6-(2-chlorphenyl)-7-[2-(1,1,1-trifluor)propylamino]-[1,2,4]triazolo[1,5-*a*]pyrimidin
5-Chlor-6-(2,4,6-trifluorphenyl)-7-[N-(2,2,2-trifluorethyl)-N-allylamino]-[1,2,4]triazolo[1,5-*a*]pyrimidin
5-Chlor-6-(2-chlor-6-fluorphenyl)-7-[N-(2,2,2-trifluorethyl)-N-allylamino]-[1,2,4]triazolo[1,5-*a*]pyrimidin
5-Chlor-6-(2-fluorphenyl)-7-[N-(2,2,2-trifluorethyl)-N-allylamino]-[1,2,4]triazolo[1,5-*a*]pyrimidin
5-Chlor-6-(2,6-difluorphenyl)-7-[N-(2,2,2-trifluorethyl)-N-allylamino]-[1,2,4]triazolo[1,5-*a*]pyrimidin
5-Chlor-6-(2-chlorphenyl)-7-[N-(2,2,2-trifluorethyl)-N-allylamino]-[1,2,4]triazolo[1,5-*a*]pyrimidin
5-Chlor-6-(2-methylphenyl)-7-[N-(2,2,2-trifluorethyl)-N-allylamino]-[1,2,4]triazolo[1,5-*a*]pyrimidin
5-Chlor-6-(2-fluorphenyl)-7-[N-(2,2,2-trifluorethyl)-N-ethylamino]-[1,2,4]triazolo[1,5-*a*]pyrimidin
5-Chlor-6-(2,6-difluorphenyl)-7-[N-(2,2,2-trifluorethyl)-N-ethylamino]-[1,2,4]triazolo[1,5-*a*]pyrimidin
5-Chlor-6-(2,4,6-trifluorphenyl)-7-[N-(2,2,2-trifluorethyl)-N-ethylamino]-[1,2,4]triazolo[1,5-*a*]pyrimidin
5-Chlor-6-(2-chlor-6-fluorphenyl)-7-[N-(2,2,2-trifluorethyl)-N-ethylamino]-[1,2,4]triazolo[1,5-*a*]pyrimidin
5-Chlor-6-(2-chlorphenyl)-7-[N-(2,2,2-trifluorethyl)-N-ethylamino]-[1,2,4]triazolo[1,5-*a*]pyrimidin
5-Chlor-6-(2-methylphenyl)-7-[N-(2,2,2-trifluorethyl)-N-ethylamino]-[1,2,4]triazolo[1,5-*a*]pyrimidin
5-Chlor-6-(2-chlor-6-fluorphenyl)-7-[N-(2,2,2-trifluorethyl)-N-(2-methylpropyl)amino]-[1,2,4]triazolo[1,5-*a*]pyrimidin
5-Chlor-6-(2,6-difluorphenyl)-7-[N-(2,2,2-trifluorethyl)-N-(2-methylpropyl)amino]-[1,2,4]triazolo[1,5-*a*]pyrimidin
5-Chlor-6-(2-fluorphenyl)-7-[N-(2,2,2-trifluorethyl)-N-(2-methylpropyl)amino]-[1,2,4]triazolo[1,5-*a*]pyrimidin
5-Chlor-6-(2,4,6-trifluorphenyl)-7-[N-(2,2,2-trifluorethyl)-N-(2-methylpropyl)amino]-[1,2,4]triazolo[1,5-*a*]pyrimidin
5-Chlor-6-(2-methylphenyl)-7-[N-(2,2,2-trifluorethyl)-N-(2-methylpropyl)amino]-[1,2,4]triazolo[1,5-*a*]pyrimidin
5-Chlor-6-(2-fluorphenyl)-7-[N-(2,2,2-trifluorethyl)-N-methylamino]-[1,2,4]triazolo[1,5-*a*]pyrimidin
5-Chlor-6-(2,6-difluorphenyl)-7-[N-(2,2,2-trifluorethyl)-N-methylamino]-[1,2,4]triazolo[1,5-*a*]pyrimidin
5-Chlor-6-(2,4,6-trifluorphenyl)-7-[N-(2,2,2-trifluorethyl)-N-methylamino]-[1,2,4]triazolo[1,5-*a*]pyrimidin
5-Chlor-6-(2-methylphenyl)-7-[N-(2,2,2-trifluorethyl)-N-methylamino]-[1,2,4]triazolo[1,5-*a*]pyrimidin
5-Chlor-6-(2-fluorphenyl)-7-[N-(2,2,2-trifluorethyl)-N-isopropylamino]-[1,2,4]triazolo[1,5-*a*]pyrimidin
5-Chlor-6-(2,6-difluorphenyl)-7-[N-(2,2,2-trifluorethyl)-N-isopropylamino]-[1,2,4]triazolo[1,5-*a*]pyrimidin
5-Chlor-6-(2,4,6-trifluorphenyl)-7-[N-(2,2,2-trifluorethyl)-N-isopropylamino]-[ 1,2,4]triazolo[1,5-*a*]pyrimidin
5-Chlor-6-(2-chlor-6-fluorphenyl)-7-[N-(2,2,2-trifluorethyl)-N-isopropylamino]-[1,2,4]triazolo[1,5-*a*]pyrimidin
5-Chlor-6-(2,6-difluorphenyl)-7-[N-(2,2,2-trifluorethyl)-N-(1-phenylethyl)amino]-[1,2,4]triazolo[1,5-*a*]pyrimidin
5-Chlor-6-(2-fluorphenyl)-7-[N,N-di-(2,2,2-trifluorethyl)amino]-[1,2,4]triazolo[1,5-*a*]pyrimidin
5-Chlor-6-(2-methylphenyl)-7-[N,N-di-(2,2,2-trifluorethyl)amino]-[1,2,4]triazolo[1,5-*a*]pyrimidin
5-Chlor-6-(2-chlorphenyl)-7-[N,N-di-(2,2,2-trifluorethyl)amino]-[1,2,4]triazolo[1,5-*a*]pyrimidin
5-Chlor-6-(2-chlor-6-fluorphenyl)-7-[N,N-di-(2,2,2-trifluorethyl)amino]-[1,2,4]triazolo[1,5-*a*]pyrimidin
5-Chlor-6-(2,6-difluorphenyl)-7-[N,N-di-(2,2,2-trifluorethyl)amino]-[1,2,4]triazolo[1,5-*a*]pyrimidin
5-Chlor-6-(2,4,6-trifluorphenyl)-7-[N,N-di-(2,2,2-trifluorethyl)amino]-[1,2,4]triazolo[1,5-*a*]pyrimidin
5-Chlor-6-(2,6-difluorphenyl)-7-[N-(2,2,2-trifluorethyl)-N-( 1,2-dimethylpropyl)amino]-[ 1,2,4]triazolo[1,5-*a*]pyrimidin
5-Chlor-6-(2,4,6-trifluorphenyl)-7-[N-(2,2,2-trifluorethyl)-N-(trimethylsilylmethyl)amino]-[1,2,4]triazolo[1,5-*a*] pyrimidin
5-Chlor-6-(2-brom-5-chlorphenyl)-7-[2-(1,1,1-trifluor)propylamino]-[1,2,4]triazolo[1,5-*a*]pyrimidin
5-Chlor-6-(2-chlorphenyl)-7-(2,2,2-trifluorethylamino)-[1,2,4]triazolo[1,5-*a*]pyrimidin
5-Chlor-6-(4-chlorphenyl)-7-(2,2,2-trifluorethylamino)-[1,2,4]triazolo[1,5-*a*]pyrimidin
5-Chlor-6-(4-bromphenyl)-7-(2,2,2-trifluorethylamino)-[1,2,4]triazolo[1,5-*a*]pyrimidin
5-Chlor-6-(4-methoxyphenyl)-7-(2,2,2-trifluorethylamino)-[1,2,4]triazolo[1,5-*a*]pyrimidin
5-Chlor-6-(4-nitrophenyl)-7-(2,2,2-trifluorethylamino)-[1,2,4]triazolo[1,5-*a*]pyrimidin
5-Chlor-6-(2-fluorphenyl)-7-(1-phenyl-2,2,2-trifluorethylamino)-[1,2,4]triazolo[1,5-*a*]pyrimidin
5-Chlor-6-(2-chlor-6-fluorphenyl)-7-[2-(1,1,1-trifluor)butylamino]-[1,2,4]triazolo[1,5-*a*]pyrimidin
5-Chlor-6-(2-chlor-6-fluorphenyl)-7-(1-phenyl-2,2,2-trifluorethylamino)-[1,2,4]triazolo[1,5-*a*]pyrimidin
5-Chlor-6-(2-fluorphenyl)-7-[2-(1,1,1 -trifluor)butylamino]-[1,2,4]triazolo[1,5-*a*]pyrimidin
5-Chlor-6-(2,6-difluorphenyl)-7-(1-phenyl-2,2,2-trifluorethylamino)-[1,2,4]triazolo[1,5-*a*]pyrimidin
5-Chlor-6-(2,4,6-trifluorphenyl)-7-(1-phenyl-2,2,2-trifluorethylamino)-[1,2,4]triazolo[1,5-*a*]pyrimidin

5-Chlor-6-(2,4,6-trifluorphenyl)-7-[2-(1,1,1-trifluor)butylamino]-[1,2,4]triazolo[1,5-*a*]pyrimidin
5-Chlor-6-(2,6-difluorphenyl)-7-[2-(1,1,1-trifluor)butylamino]-[1,2,4]triazolo[1,5-*a*]pyrimidin
5-Chlor-6-(2-chlorphenyl)-7-[2-(1,1,1-trifluor)butylamino]-[1,2,4]triazolo[1,5-*a*]pyrimidin
5-Chlor-6-(2-chlorphenyl)-7-(1-phenyl-2,2,2-trifluorethylamino)-[1,2,4]triazolo[1,5-*a*]pyrimidin
5-Chlor-6-(2,4-difluorphenyl)-7-(2,2,2-trifluorethylamino)-[1,2,4]triazolo[1,5-*a*]pyrimidin
5-Chlor-6-(2,4,6-trifluorphenyl)-7-[2-(1,1,1-trifluor)-3-methylbutylamino]-[1,2,4]triazolo[1,5-*a*]pyrimidin
5-Chlor-6-(2,6-difluor-4-methoxyphenyl)-7-[2-(1,1,1-trifluor)propylamino]-[1,2,4]triazolo[1,5-*a*]pyrimidin

7. Verfahren zur Herstellung einer Verbindung der Formel I nach einem der vorhergehenden Ansprüche, **dadurch gekennzeichnet, daß** man eine Verbindung der allgemeinen Formel II

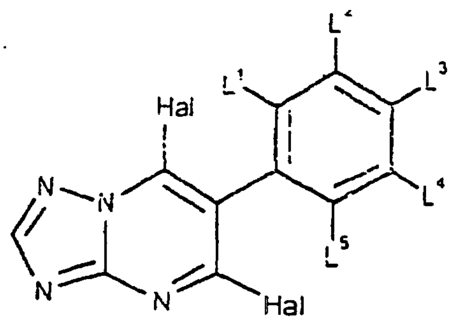

(II)

in der
$L^1$ bis $L^5$ und Hal nach einem der vorgehenden Ansprüche definiert sind,
mit einem Amin der allgemeinen Formel III

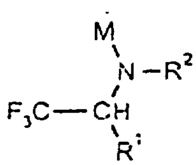

(III)

in der
$R^1$ und $R^2$ nach einem der vorhergehenden Ansprüche definiert sind und
M ein Wasserstoffatom oder ein freies oder komplexiertes Metallatom bedeutet, behandelt, wodurch man zu einer Verbindung der Formel I gelangt.

8. Fungizide Zusammensetzung enthaltend einen Träger sowie mindestens einer Verbindung der Formel I nach einem der Ansprüche 1 bis 6 als Wirkstoff.

9. Verfahren zur Bekämpfung von phytopathogenen Pilzen an einem Standort, bei dem man den Standort mit einer Verbindung der Formel I nach einem der Ansprüche 1 bis 6 oder mit einer Zusammensetzung nach Anspruch 8 behandelt.

10. Verwendung einer Verbindung der Formel I nach einem der Ansprüche 1 bis 6 oder einer Zusammensetzung nach Anspruch 8 als Fungizid.

**Revendications**

1. Composé de formule générale I

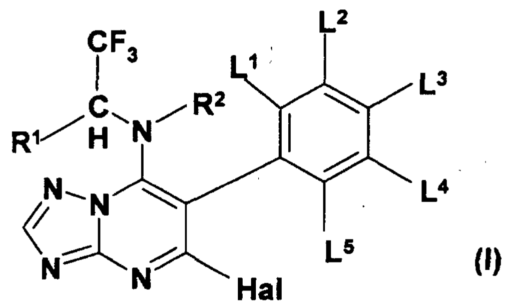

(I)

dans lequel

$R^1$ et $R^2$ représentent chacun indépendamment un atome d'hydrogène ou un groupe alkyle, alcényle, alcynyle, alcadiényle ou phényle, éventuellement substitué,
Hal représente un atome d'halogène
$L^1$ à $L^5$ représentent chacun indépendamment un atome d'hydrogène ou d'halogène ou un groupe alkyle, alcoxy ou nitro

dans lequel
les termes alkyle, alcényle, alcynyle, alcadiényle désignent des radicaux ou fractions à chaîne droite ou ramifiée ayant jusqu'à 10 atomes de carbone;
dans lequel
chaque groupe éventuellement substitué est substitué, indépendamment par un ou plusieurs atomes d'halogène ou des groupes nitro, cyano, hydroxy, alkyle, cycloalkyle, cycloalcényle, halogénoalkyle, halogénocycloalkyle, alcoxy, halogénoalcoxy, trialkylsilyle, phényle, halogéno- ou dihalogéno-phényle ou pyridyle.

**2.** Composé selon la revendication 1, dans lequel au moins l'un de $L^1$ et de $L^5$ représentent un atome d'halogène.

**3.** Composé selon la revendication 1 ou 2, dans lequel $R^1$ représente un atome d'hydrogène ou un groupe méthyle.

**4.** Composé selon l'une quelconque des revendications précédentes, dans lequel $R^2$ représente un atome d'hydrogène ou un groupe alkyle en $C_1$ à $C_{10}$.

**5.** Composé selon l'une quelconque des revendications précédentes, dans lequel au moins l'un de $R^1$ et de $R^2$ représente un atome d'hydrogène.

**6.** Composés de formule I suivants:

la 5-chloro-6-(2-chloro-6-fluorophényl)-7-(2,2,2-trifluoroéthylamino)-[1,2,4]triazolo[1,5-*a*]pyrimidine,
la 5-chloro-6-(2,6-difluorophényl)-7-(2,2,2-trifluoroéthylamino)-[1,2,4]triazolo[1,5-*a*]pyrimidine,
la 5-chloro-7-(2,2,2-trifluoroéthylamino)-6-(2,4,6-trifluorophényl)-[1,2,4]triazolo[1,5-*a*]pyrimidine,
la 5-chloro-6-(2-chlorophényl)-7-(2,2,2-trifluoroéthylamino)-[1,2,4]triazolo[1,5-*a*]pyrimidine,
la 5-chloro-6-(2-fluorophényl)-7-(2,2,2-trifluoroéthylamino)-[1,2,4]triazolo[1,5-*a*]pyrimidine,
la 5-chloro-6-(2-chloro-6-fluorophényl)-7-[2-(1,1,1-trifluoro)propylamino]-[1,2,4]triazolo[1,5-*a*]pyrimidine,
la 5-chloro-6-(2,6-difluorophényl)-7-[2-(1,1,1-trifluoro)propylamino]-[1,2,4]triazolo[1,5-*a*]pyrimidine,
la 5-chloro-6-(2,4,6-trifluorophényl)-7-[2-(1,1,1-trifluoro)propylamino]-[1,2,4]triazolo[1,5-*a*]pyrimidine,
la 5-chloro-6-(2-méthylphényl)-7-[2-(1,1,1-trifluoro)propylamino]-[1,2,4]triazolo[1,5-*a*]pyrimidine,
la 5-chloro-6-(2-fluorophényl)-7-[2-(1,1,1-trifluoro)propylamino]-[1,2,4]triazolo[1,5-*a*]pyrimidine,
la 5-chloro-6-(2-chlorophényl)-7-[2-(1,1,1-trifluoro)propylamino]-[1,2,4]triazolo[1,5-*a*]pyrimidine,
la 5-chloro-6-(2,4,6-trifluorophényl)-7-[N-(2,2,2-trifluoroéthyl)-N-allylamino]-[1,2,4]triazolo[1,5-*a*]pyrimidine,
la 5-chloro-6-(2-chloro-6-fluorophényl)-7-[N-(2,2,2-trifluoroéthyl)-N-allylamino]-[1,2,4]triazolo[1,5-*a*]pyrimidine,
la 5-chloro-6-(2-fluorophényl)-7-[N-(2,2,2-trifluoroéthyl)-N-allylamino]-[1,2,4]triazolo[1,5-*a*]pyrimidine,
la 5-chloro-6-(2,6-difluorophényl)-7-[N-(2,2,2-trifluoroéthyl)-N-allylamino]-[1,2,4]triazolo[1,5-*a*]lpyrimidine,
la 5-chloro-6-(2-chlorophényl)-7-[N-(2,2,2-trifluoroéthyl)-N-allylamino]-[1,2,4]triazolo[1,5-*a*]pyrimidine,

la 5-chloro-6-(2-méthylphényl)-7-[N-(2,2,2-trifluoroéthyl)-N-allylamino]-[1,2,4]triazolo[1,5-*a*]pyrimidine,
la 5-chlore-6-(2-fluorophényl)-7-[N-(2,2,2-trilluoroéthyl)-N-éthylamino]-[1,2,4]triazolo[1,5-*a*]pyrimidine,
la 5-chloro-6-(2,6-difluorophényl)-7-[N-(2,2,2-trifluoroéthyl)-N-éthylamino]-[1,2,4]triazolo[1,5-*a*]pyrimidine,
la 5-chloro-6-(2, 4, 6, trifluorophényl)-7-[N-(2,2,2-trifluoroéthyl)-N-éthylamino]-[1,2,4]triazolo[1,5-*a*]pyrimidine,
la 5-chloro-6-(2-chloro-6-fluorophényl)-7-[N-(2,2,2-trifluoroéthyl)-N-éthylamino]-[1,2,4]triazolo[1,5-*a*]pyrimidine,
la 5-chloro-6-(2-chlorophényl)-7-[N-(2,2,2-trifluoroéthyl)-N-éthylamino]-[1,2,4]triazolo[1,5-*a*]pyrimidine,
la 5-chloro-6-(2-méthylphényl)-7-[N-(2,2,2-trifluoroéthyl)-N-éthylamino]-[1,2,4]triazolo[1,5-*a*]pyrimidine,
la 5-chloro-6-(2-chloro-6-fluorophényl)-7-[N-(2,2,2-trifluoroéthyl)-N-(2-méthylpropyl)amino]-[1,2,4]triazolo[1,5-*a*]pyrimidine,
la 5-chloro-6-(2,6-difluorophényl)-7-[N-(2,2,2-trifluoroéthyl)-N-(2-méthylpropyl)amino]-[1,2,4]triazolo[1,5-*a*]pyrimidine,
la 5-chlore-6-(2-fluorophényl)-7-[N-(2,2,2-trifluoroéthyl)-N-(2-méthyl(propyl)-amino][1,2,4]triazolo[1,5-*a*]pyrimidine,
la 5-chloro-6-(2,4,6-trifluorophényl)-7-[N-(2,2,2-trifluoroéthyl)-N-(2-méthylpropyl)amino]-[1,2,4]triazolo[1,5-*a*]pyrimidine,
la 5-chloro-6-(2-méthylphényl)-7-[N-(2,2,2-trifluoroéthyl)-N-(2-méthylpropyl)-amino][1,2,4]triazolo[1,5-*a*]pyrimidine,
la 5-chloro-6-(2-fluorophényl)-7-[N-(2,2,2-trifluoroéthyl)-N-méthylamino]-[1,2,4]triazolo[1,5-*a*]pyrimidine,
la 5-chloro-6-(2,6-difluorophényl)-7-[N-(2,2,2-trifluoroéthyl)-N-méthylamino]-[1,2,4]triazolo[1,5-*a*]pyrimidine,
la 5-chloro-6-(2,4,6-trifluorophényl)-7-[N-(2,2,2-trifluoroéthyl)-N-méthylamino]-[1,2,4]triazolo[1,5-*a*]pyrimidine,
la 5-chlore-6-(2-méthylphényl)-7-[N-(2,2,2-trifluoroéthyl)-N-méthylamino]-[1,2,4]triazolo[1,5-*a*]pyrimidine,
la 5-chloro-6-(2-fluorophényl)-7-[N-(2,2,2-trifluoroéthyl)-N-isopropylamino]-[1,2,4]triazolo[1,5-*a*]pyrimidine,
la 5-chloro-6-(2,6-difluorophényl)-7-[N-(2,2,2-trifluoroéthyl)-N-isopropylamino]-[1,2,4]triazolo[1,5-*a*]pyrimidine,
la 5-chloro-6-(2,4,6-trifluorophényl)-7-[N-(2,2,2-trifluoroéthyl)-N-isopropylamino]-[1,2,4]triazolo[1,5-*a*]pyrimidine,
la 5-chloro-6-(2-chloro-6-fluorophényl)-7-[N-(2,2,2-trifluoroéthyl)-N-isopropylamino][1,2,4]triazolo[1,5-*a*]pyrimidine,
la 5-chloro-6-(2,6-difluorophényl)-7-[N-(2,2,2-trifluoroéthyl)-N-(1phényléthyl)-amino]-[1,2,4]triazolo[1,5-*a*]pyrimidine,
la 5-chloro-6-(2-fluorophényl)-7-[N,N-di-(2,2,2-trifluoroéthyl)amino]-[1,2,4]triazolo[1,5-*a*]pyrimidine,
la 5-chloro-6-(2-méthylphenyl)-7-[N,N-di-(2,2,2-trifluoroéthyl)amino]-[1,2,4]triazolo[1,5-*a*]pyrimidine,
la 5-chloro-6-(2-chlorophényl)-7-[N,N-di-(2,2,2-trifluoroéthyl)amino]-[1,2,4]triazolo[1,5-*a*]pyrimidine,
la 5-chloro-6-(2-chloro-6-fluorophényl)-7-[N,N-di-(2,2,2-trifluoroéthyl)amino]-[1,2,4]triazolo[1,5-*a*]pyrimidine,
la 5-chloro-6-(2,6-difluorophényl)-7-[N,N-di-(2,2,2-trifluoroéthyl)amino]-[1,2,4]triazolo[1,5-*a*]pyrimidine,
la 5-chloro-6-(2,4,6-trifluorophényl)-7-[N,N-di-(2,2,2-trifluoroéthyl)amino]-[1,2,4]triazolo[1, 5-a]pyrimidine,
la 5-chloro-6-(2,6-difluorophényl)-7-[N,-(2,2,2-trifluoroéthyl)-N-(1,2-diméthylpropyl)amino]-[1,2,4]triazolo[1,5-*a*]pyrimidine,
la 5-chloro-6-(2,4,6-trifluorophényl)-7-[N,-(2,2,2-trifluoroéthyl)-N(triméthylsilylméthyl)amino]-[1,2,4]triazolo[1,5-*a*]pyrimidine,
la 5-chloro-6-(2-bromo-5-chlorophényl)-7-[2-(1,1,1-trifluoro)propylamino]-[1,2,4]triazolo[1,5-*a*]pyrimidine,
la 5-chloro-6-(2-chlorophényl)-7-(2,2,2-trifluoroéthylamino)-[1,2,4]triazolo[1,5-*a*]pyrimidine,
la 5-chloro-6-(4-chlorophényl)-7-(2,2,2-trifluoroéthylamino)-[1,2,4]triazolo[1,5-*a*]pyrimidine,
la 5-chloro-6-(4-bromophényl)-7-(2,2,2-trifluoroéthylamino)-[1,2,4]triazolo[1,5-*a*]pyrimidine,
la 5-chloro-6-(4-méthoxyphényl)-7-(2,2,2-trifluoroéthylamino)-[1,2,4]triazolo[1,5-*a*]pyrimidine,
la 5-chloro-6-(4-nitrophényl)-7-(2,2,2-trifluoroéthylamino)-[1,2,4]triazolo[1,5-*a*]pyrimidine,
la 5-chloro-6-(2-fluorophényl)-7-(1-phényl-2,2,2-trifluoroéthylamino)-[1,2,4]triazolo[1,5-*a*]pyrimidine,
la 5-chloro-6-(2-chloro-6-fluorophényl)-7-[2-(1,1,1-trifluoro)butylamino]-[1,2,4]triazolo[1,5-*a*]pyrimidine,
la 5-chloro-6-(2-chloro-6-fluorophényl)-7-(1-phényl-2,2,2-trifluoroéthylamino)-[1,2,4]triazolo[1,5-*a*]pyrimidine,
la 5-chloro-6-(2-fluorophényl)-7-[2-(1,1,1-trifluoro)butylamino]-[1,2,4]triazolo[1,5-*a*]pyrimidine,
la 5-chloro-6-(2,6-difluorophényl)-7-(1-phényl-2,2,2-trifluoroéthylamino)-[1,2,4]triazolo[1,5-*a*]pyrimidine,
la 5-chloro-6-(2,4,6-trifluorophényl)-7-(1-phényl-2,2,2-trifluoroéthylamino)-[1,2,4]triazolo[1,5-*a*]pyrimidine,
la 5-chloro-6-(2,4,6-trifluorophényl)-7-[2-(1,1,1 -trifluoro)butylamino]-[1,2,4]triazolo[1,5-*a*]pyrimidine,
la 5-chloro-6-(2,6-difluorophényl)-7-[2-(1,1,1-trifluoro)butylamino]-[1,2,4]triazolo[1,5-*a*]pyrimidine,
la 5-chloro-6-(2-chlorophényl)-7-[2-(1,1,1-trifluoro)butylamino]-[1,2,4]triazolo[1,5-*a*]pyrimidine,
la 5-chloro-6-(2-chlorophényl)-7-(1-phényl-2,2,2-trifluoroéthylamino)-[1,2,4]triazolo[1,5-*a*]pyrimidine,

la 5-chloro-6-(2,4-difluorophényl)-7-(2,2,2-trifluoroéthylamino)-[1,2,4]triazolo[1,5-*a*]pyrimid ine,
la 5-chloro-6-(2,4,6-trifluorophényl)-7-[2-(1,1,1-trifluoro)-3-méthylbutylamino]-[1,2,4]triazolo[1,5-*a*]pyrimidine,
la 5-chloro-6-(2,6-difluoro-4-méthoxyphényl)-7-[2-(1,1,1-trifluoro)propylamino]-[1,2,4]triazolo[1,5-*a*]pyrimidine.

**7.** Procédé de préparation d'un composé de formule I selon l'une quelconque des revendications précédentes qui comprend le traitement d'un composé de formule générale II

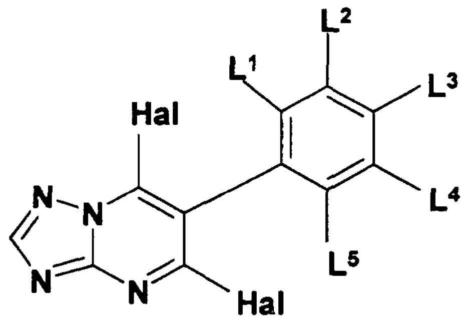

(II)

dans lequel
$L^1$ à $L^5$ et Hal sont tels que définis selon l'une quelconque des revendications précédentes, avec une amine de formule général III

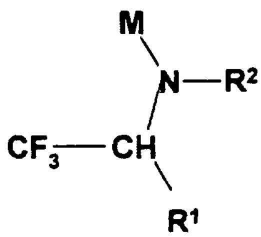

(III)

dans lequel
$R^1$ et $R^2$ sont tels que définis selon l'une quelconque des revendications précédentes,
M représente un atome d'hydrogène ou un atome métallique libre ou complexé,
afin de produire un composé de formule 1.

**8.** Composition fongicide qui comprend un véhicule, et comme agent actif, au moins un composé de formule 1 selon l'une quelconque des revendications 1 à 6.

**9.** Procédé de lutte contre un champignon phytopathogène en un lieu, qui comprend le traitement du lieu avec un composé de formule 1 selon l'une quelconque des revendications 1 à 6 ou avec une composition selon la revendication 8.

**10.** Utilisation en tant que fongicide, d'un composé de formule I selon l'une quelconque des revendications 1 à 6 ou d'une composition selon la revendication 8.